# EUROPEAN PATENT APPLICATION

(11) **EP 4 044 211 A1**
(43) Date of publication of application: **17.08.2022**
(21) Application number: 21156663.3
(22) Date of filing: 11.02.2021
(51) Int. Cl.: H01J 49/04, H01J 49/16

(54) **MALDI-MS METHOD, PHOTO-SENSITIVE MALDI MATRIX COMPOSITE AND PHOTO-CAGED MALDI MATRIX COMPOUND FOR USE IN SAID METHOD AND RESPECTIVE USES**

(71) Applicant: Sirius Fine Chemicals SiChem GmbH, 28359 Bremen (DE)
(72) Inventor: Hopf, Carsten, 68165 Mannheim (DE); Gillandt, Hartmut, 28359 Bremen (DE)
(74) Representative: Eisenführ Speiser

(57) **Abstract**

Described herein is a Photo-Sensitive MALDI Matrix Composite for use in a method of matrix-assisted laser desorption/ionization mass spectrometry and the use of a Photo-caged MALDI Matrix Compound as matrix compound in a method of matrix-assisted laser desorption/ionization mass spectrometry. Moreover, a matrix-assisted laser desorption/ionization mass spectrometry method involving the Photo-Sensitive MALDI Matrix Composite and the Photo-Caged MALDI Matrix Compound is described as well as a Photo-Caged MALDI Matrix Compound.

## Description

The present invention relates to a Photo-sensitive MALDI Matrix Composite for use in a method of matrix-assisted laser desorption/ionization mass spectrometry and to the use of a Photo-caged MALDI Matrix Compound as matrix compound in a method of matrix-assisted laser desorption/ionization mass spectrometry. Moreover, the present invention pertains to a matrix-assisted laser desorption/ionization mass spectrometry method involving the Photo-sensitive MALDI Matrix Composite and the Photo-caged MALDI Matrix Compound as well as to a Photo-caged MALDI Matrix Compound.

Matrix-assisted laser desorption/ionization (MALDI) mass spectrometry (MS) is an ionization technique that uses a laser energy-absorbing matrix to create ions from large molecules with minimal fragmentation. MALDI-MS has been used for the analysis of larger molecules, including biomolecules, e.g. biopolymers such as nucleic acids, proteins, peptides or polysaccharides, or of other organic molecules, including polymers, dendrimers or certain macromolecules, in particular such larger molecules which tend to be fragile and to fragment when ionized by other, less gentle ionization methods. Today, MALDI-MS and its variants have developed into extremely valuable analytical tools for clinical and pharmaceutical research.

The conventional MALDI-MS method usually comprises three steps, i.e. (i) mixing a sample to be analysed (usually referred to as the "analyte") with a suitable matrix material (also referred to as "MALDI matrix compound"), thereby embedding the analyte in the matrix material, ideally including a step of co-crystallizing the analyte with the matrix material, and applying the matrix material with the embedded analyte (together also referred to herein as a "MALDI matrix composite") to an object slide, (ii) irradiating the matrix material with the embedded analyte with a pulsed laser to trigger ablation and desorption thereof from the object slide, usually including creation of a gas phase, and (iii) ionizing the analyte molecules, presumably by protonation or deprotonation in the hot plume of ablated gases. Subsequently, the ionized analyte molecules, in the gas phase, are provided to the inlet of a mass spectrometer.

MALDI-MS is increasingly used today in high-throughput screening processes where larger molecular libraries need to be analysed within relatively short time periods. This application sets high demands on automatization techniques, robust test methods and analysis of results.

MALDI mass spectrometry imaging (MALD-MSI) is a more specific application of MALDI-MS, i.e. a technique for visualization of the spatial distribution of small molecular and macromolecular biomolecules in tissue sections. MALDI-MSI is often used in pharmaceutical analytics and facilitates an in-depth analysis of drug compounds, metabolites, adjuvants and contaminants in a tissue sample. In related analytical methods, about 200.000 measuring points need to be scanned with a step size of about 20 µm over a period of one hour. It is planned for future applications to further reduce the distance between measuring points by a factor of ten, which means an increase of measuring points by a factor of 100 and to measure entire (clinical) cohorts consisting of 100 and more such samples, which also means the increase of measuring points by a factor of 10. For realizing such ambitious goals, MALDI-MS or MALDI-MSI measuring cycle times of up to 72 hours or more will be required.

Since MALDI techniques usually involve applying a vacuum, a MALDI matrix compound suitable for future high-performance MALDI-MS or MALDI-MSI analytical methods, e.g. in processes for serial imaging of multiple tissue sections - like entire clinical cohorts - with high spatial resolution, will need to be stable under such vacuum conditions over extended time periods, e.g. for time periods of about 72 hours or more, as explained above, and not prematurely evaporate. However, many MALDI matrix compounds known today do usually not possess the required long-term stability against evaporation under vacuum.

Correspondingly, it was a primary object of the present invention to provide a MALDI matrix composite which would provide for increased vacuum stability, e.g. to allow extended measuring cycles in MALDI-MS or MALDI-MSI methods.

It was another object of the present invention to provide a suitable MALDI matrix compound for preparing said MALDI matrix composite with increased stability against evaporation upon exposure to a vacuum.

It was a further object of the present invention to provide a MALDI-MS or MALDI-MSI method which would make use of a MALDI matrix composite and/or a MALDI matrix composite with increased stability against evaporation upon exposure to a vacuum, e.g. to allow for extended measuring cycles.

It has now been found that the primary object and other objects of the present invention can be accomplished by a Photo-sensitive MALDI Matrix Composite for use in a method of matrix-assisted laser desorption/ionization mass spectrometry, comprising or consisting of
C1) a matrix, completely or partially constituted (preferably completely constituted) by a Photo-caged MALDI Matrix Compound, comprising a Photoremovable Protecting Group Moiety B which is covalently bonded to an organic MALDI Matrix Compound Moiety A
   and embedded in said matrix
C2) one or more than one analyte, to be analyzed in the method of matrix-assisted laser desorption/ionization mass spectrometry.

The invention as well as preferred variants and preferred combinations of parameters, properties and elements thereof are defined in the appended claims. Preferred aspects, details, modifications and advantages of the present invention are also defined and explained in the following description and in the examples shown below.

In the context of the present invention, the meaning of the term "matrix-assisted laser desorption/ionization mass spectrometry", in accordance with the usual meaning in the field, comprises the methods of MALDI-MS, MALDI-MSI and "MALDI-2" as are generally known in the field. "MALDI-2" is a more sensitive MALDI-MS method involving laser-induced postionization of the analyte and/or the MALDI matrix compound, to enhance mass spectrometry imaging of numerous classes of biomolecules, as e.g. described by J. Soltwisch et al. in SCIENCE, Vol. 384, Issue 6231 (2015), pp. 211-215 or by J. Soltwisch et al. in Anal. Chem. 92, 13 (2020) pp. 8697-8703.

In component C1) of the Photo-sensitive MALDI Matrix Composite according to the present invention as defined above, the matrix may be completely or partially constituted by a Photo-caged MALDI Matrix Compound. For example, the matrix can be completely constituted by a Photo-caged MALDI Matrix Compound or the matrix can be partially constituted by a Photo-caged MALDI Matrix Compound and partially by a MALDI matrix compound as is known in the art. In the latter case, the matrix can in particular be partially constituted by a Photo-caged MALDI Matrix Compound and partially by a MALDI matrix compound which comprises an equal organic MALDI Matrix Compound Moiety A as is also part of the Photo-caged MALDI Matrix Compound constituting the same matrix.

Preferred is a Photo-sensitive MALDI Matrix Composite according to the present invention as described herein (or a Photo-sensitive MALDI Matrix Composite according to the present invention as described herein as preferred) wherein
- the one or at least one of the more than one analyte is selected from the group consisting of proteins, peptides, lipids, nucleic acids, polysaccharides, glycopeptides, organometallic compounds and organic polymers, wherein preferably the molar mass of the analyte is in the range of from ≥ 100 Da to ≤ 200,000 Da, more preferably of from ≥ 100 Da to ≤ 50,000 Da and even more preferably of from ≥ 100 Da to ≤ 15,000 Da;
   and/or
- the total mass ratio of Photo-caged MALDI Matrix Compound : analyte is in the range of from ≥ 100 : 1 to ≤ 20,000 : 1, preferably of ≥ 500 : 1 to ≤ 10,000 : 1;
   and/or
- the Photo-sensitive MALDI Matrix Composite is present in the solid state at 23 °C and 101,3 kPa;
   and/or
- the Photo-caged MALDI Matrix Compound is photo-cleavable, preferably photo-cleavable at the covalent bond (or at a covalent bond) between its moieties A and B, upon irradiation with radiation of a wavelength in the range of from ≥ 100 nm to ≤ 15 µm, preferably of from ≥ 150 nm to ≤ 12 µm, wherein preferably the radiation is or comprises radiation from a laser source, more preferably from a pulsed laser source and even more preferably from a modulated pulsed laser source.

The Photo-caged MALDI Matrix Compound present in the Photo-sensitive MALDI Matrix Composite has a higher molecular mass and a lower vapor pressure (at 23 °C) than the corresponding MALDI matrix compound which can be released from it by photo-cleavage. This has the effect that said MALDI matrix compound is less stable against evaporation when exposed to a vacuum than the corresponding Photo-caged MALDI Matrix Compound from which it can be released by photo-cleavage. A Photo-sensitive MALDI Matrix Composite according to the present invention is therefore particularly stable for extended time periods under vacuum conditions as are usually applied in methods of matrix-assisted laser desorption/ionization mass spectrometry like MALDI-MS or MALDI-MSI.

Where in the Photo-sensitive MALDI Matrix Composite according to the present invention as defined above, the total mass ratio of Photo-caged MALDI Matrix Compound : analyte is in the range of from ≥ 100 : 1 to ≤ 20,000 : 1, the term "total mass" in this case refers to the total mass of the Photo-caged MALDI Matrix Compound or the analyte, respectively, both as present in the Photo-sensitive MALDI Matrix Composite, not their molar masses.

The Photo-sensitive MALDI Matrix Composite according to the present invention as defined above may be present at 23 °C and ambient pressure (i.e. 101,3 kPa) in the liquid state, in particular in the form of an ionic liquid matrix (e.g. as described by Y. Yamazaki et al. in J Am Soc Mass Spectrom. 2020 Jun 3; 31 (6): 1180-1188) or in the solid state. In a preferred variant of the present invention, the Photo-sensitive MALDI Matrix Composite is present in the solid state under the said conditions.

Similarly, the Photo-caged MALDI Matrix Compound as described herein (in particular the Photo-caged MALDI Matrix Compound according to the present invention as described herein) may be present at 23 °C and ambient pressure (i.e. 101,3 kPa) in the liquid state, in particular in the form of an ionic liquid (e.g. as described by Y. Yamazaki et al. in J Am Soc Mass Spectrom. 2020 Jun 3; 31 (6): 1180-1188) or in the solid state. In a preferred variant of the present invention, the Photo-caged MALDI Matrix Compound as described herein (in particular the Photo-caged MALDI Matrix Compound according to the present invention as described herein) is present in the solid state under the said conditions.

Laser sources suitable for providing the radiation for photo-cleaving the Photo-caged MALDI Matrix Compound, preferably photo-cleaving at the covalent bond between its moieties A and B comprise common lasers known in the art. Such lasers include UV lasers, in particular nitrogen lasers (operating at a wavelength of 337 nm), frequency-tripled and quadrupled neodymium-doped yttrium aluminum garnet lasers (Nd:YAG lasers, operating at a wavelength of 355 nm or 266 nm, respectively); infrared (IR) lasers, in particular erbium-doped yttrium aluminum garnet lasers (Er:YAG lasers, operating at a wavelength of 2.94 µm), mid-IR optical parametric oscillators and carbon dioxide lasers (operating at a wavelength of 10.6 µm). Diode lasers are also suited for the purpose explained above. Particularly suited examples of lasers for providing the radiation for photo-cleaving the Photo-caged MALDI Matrix Compound are modulated Bruker Smartbeam^{™} Lasers as described e.g. by A. Holle et al. in "J. Mass Spectrom." 41 (2006) 705 - 716.

Also preferred is a Photo-sensitive MALDI Matrix Composite according to the present invention as described herein (or a Photo-sensitive MALDI Matrix Composite according to the present invention as described herein as preferred), wherein the Photo-caged MALDI Matrix Compound present in the Photo-sensitive MALDI Matrix Composite comprises
M1) an organic MALDI Matrix Compound Moiety A comprising
   - two or more than two conjugated double bonds in its chemical structure,
   - one or more than one heteroatom in its chemical structure, preferably selected from the group consisting of nitrogen, oxygen and sulfur, more preferably selected from the group consisting of nitrogen and oxygen,
   and preferably having
   - a molar mass of 500 g/mol or less;
   and
M2) a Photoremovable Protecting Group Moiety B,
   wherein the Photoremovable Protecting Group Moiety B is covalently bonded to the one or to one of the more than one heteroatoms of the organic MALDI Matrix Compound Moiety A,
   wherein preferably the covalent bond between the Photoremovable Protecting Group Moiety B and the one heteroatom, or between the Photoremovable Protecting Group Moiety B and one of the more than one heteroatoms, of the organic MALDI Matrix Compound Moiety A has a bond energy in the range of from ≥ 1 to ≤ 8 eV, preferably in the range of from ≥ 1.5 to ≤ 7 eV.

As explained above, component C1) of the Photo-sensitive MALDI Matrix Composite according to the present invention, the Photo-caged MALDI Matrix Compound, is photo-cleavable, preferably at the covalent bond (or at a covalent bond) between its moieties A and B, upon irradiation with suitable radiation of a wavelength in the range of from ≥ 100 nm to ≤ 15 µm (or a preferred wavelength range as described herein). Preferably, upon cleavage of the photo-cleavable Photo-caged MALDI Matrix Compound, the MALDI Matrix Compound Moiety A releases a MALDI matrix compound. The analyte is then preferably embedded in said MALDI matrix compound. The resulting MALDI matrix compound with the analyte embedded therein (also referred to herein as a "MALDI matrix composite") can then be used in a common method of matrix-assisted laser desorption/ionization mass spectrometry, as is known in the art.

As explained above, the organic MALDI Matrix Compound Moiety A (component C1)) preferably has a molar mass of 500 g/mol or less, more preferably in the range of from ≥ 100 g/mol to ≤ 500 g/mol, even more preferably of from ≥ 150 g/mol to ≤ 400 g/mol and yet even more preferably of from ≥ 150 g/mol to ≤ 350 g/mol.

According to the present invention, the organic MALDI Matrix Compound Moiety A comprises one or more than one heteroatom in its chemical structure. In the context of the present invention, a "heteroatom" means an atom which is not a carbon atom and which preferably can be part of the structure of an organic molecule. Preferably, the heteroatom comprised by the chemical structure of the organic MALDI Matrix Compound Moiety A is selected from the group consisting of nitrogen, oxygen and sulfur, more preferably it is selected from the group consisting of nitrogen and oxygen.

Suitable MALDI matrix compounds, from which the MALDI Matrix Compound Moiety A (as defined above) can be derived, comprise MALDI matrix compounds known from the prior art, e.g. as disclosed by R. Zenobi et al. in "Mass Spectrometry Reviews" 17 (1998) 337-366, or by Q. Zhou et al. in "Anal Bioanal Chem." 2020 (November - online ahead of print) but preferably also comprise compounds suited for the very purpose which are still to be identified.

The skilled person is aware that a substance in order to be suited as a MALDI matrix compound should preferably show one or more typical properties, comprising:
(a) a pronounced absorption in the region of commonly used UV or IR laser wavelengths, e.g. by the presence of two or more than two conjugated double bonds in the chemical structure of the MALDI matrix compound;
(b) at best cause minimal matrix background signals and adducts with analytes ("chemical noise"), especially in the low mass range (typically m/z < 500), thus enabling visualization of the spatial distribution of small molecules;
(c) an ability to interact with analyte molecules, ideally an ability of forming co-crystals with analyte molecules;
(d) an ability to effectively ionize analyte molecules, resulting in protonated ions in positive ion mode or deprotonated ions in negative ion mode.

In the context of the present invention, the term "photoremovable protecting group", in accordance with the usual meaning in the field, means a chemical group which can be attached to a chemical (usually organic) molecule and can be (preferably selectively) removed again from said chemical (usually organic) molecule with electromagnetic radiation of a wavelength in the range of from ≥ 100 nm to ≤ 15 µm, or with electromagnetic radiation of the preferred wavelength ranges as further defined herein. Common synonyms for the term "photoremovable protecting group" are "photosensitive protecting group", "photocleavable protecting group" or "photoreleasable protecting group". A variety of photoremovable protecting groups is known in the prior art, e.g. from P Klán et al. in "Chemical Reviews" 113 (2013) pp. 119-191 and literature cited therein, as well as from A. Y. Vorobev in Computational and Structural Biotechnology Journal 18 (2020) pp. 27-34 or from the "CRC Handbook of Organic Photochemistry and Photobiology", in particular chapter on "Photolabile Protecting Groups in organic Synthesis" CRC Press 3rd ed. 2012 (or previous editions of this book).

According to the present invention, a photoremovable protecting group is preferably attached to a MALDI matrix compound by (chemical modification) methods known in the art, to form a Photo-caged MALDI Matrix Compound, wherein the organic MALDI Matrix Compound Moiety A of said Photo-caged MALDI Matrix Compound is derived from said MALDI matrix compound and the Photoremovable Protecting Group Moiety B of said Photo-caged MALDI Matrix Compound is derived from said photoremovable protecting group. For example, the Photoremovable Protecting Group Moiety B of said Photo-caged MALDI Matrix Compound can in principle be derived from the photoremovable protecting groups known from the literature references cited here above.

Furthermore preferred is a Photo-sensitive MALDI Matrix Composite according to the present invention as described herein (or a Photo-sensitive MALDI Matrix Composite according to the present invention as described herein as preferred), wherein the Photoremovable Protecting Group Moiety B of the Photo-caged MALDI Matrix Compound is selected from (or is derived from photoremovable protecting groups, respectively, as explained above, selected from) the group consisting of
- arylcarbonylmethyl groups, preferably selected from the group consisting of phenacyl groups, o-alkylphenacyl groups, p-hydroxyphenacyl groups and benzoin groups;
- nitroaryl groups, preferably nitrophenyl groups;
- coumarin-4-ylmethyl groups;
- arylmethyl groups;
- a pivaloyl group;
- carbonyl cyclo(hetero)alkyl groups;
- carbonylaryl groups;
- arylsulfonyl groups;
- silyl groups;
- 2-hydroxycinnamyl groups;
- α-keto amide groups;
- α,β-unsaturated anilide groups;
- methyl(phenyl)thiocarbamic acid groups;
- thiochromone S,S-dioxide groups;
- 2-pyrrolidino-1,4-benzoquinone groups;
- triazine groups;
- arylmethyleneimino groups;
- xanthene groups
and
- pyronin groups,
   or is derived from one of the aforementioned groups;
   wherein preferably the Photoremovable Protecting Group Moiety B is selected from the group consisting of
      - arylcarbonylmethyl groups, preferably selected from the group consisting of phenacyl groups, o-alkylphenacyl groups, p-hydroxyphenacyl groups and benzoin groups;
      and
      - nitroaryl groups, preferably nitrophenyl groups
      or is derived from one of the aforementioned (preferred) groups.

In a more specific variant of the present invention is preferred a Photo-sensitive MALDI Matrix Composite according to the present invention as described herein (or a Photo-sensitive MALDI Matrix Composite according to the present invention as described herein as preferred), wherein the organic MALDI Matrix Compound Moiety A of the Photo-caged MALDI Matrix Compound is selected from the group consisting of
- a moiety of formula I-1 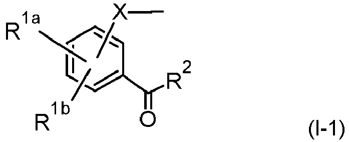 wherein
   - R^{1a} and R^{1b}: are both independently of each other selected from the group consisting of hydrogen, hydroxy, carboxy, methyl and methoxy,
   - R²: is selected from the group consisting of hydroxy and methyl and
   - X: is selected from the group consisting of oxygen (-O-), -N(H)-, -N(CH₃)- and -N(C₂H₅)-;
- a moiety of formula I-2 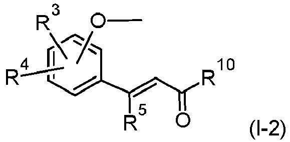 wherein
   - R³ and R⁴: are independently of each other selected from the group consisting of hydrogen, hydroxy, halogen and methoxy and
   - R⁵: is selected from the group consisting of hydroxy, methyl and cyano and
   - R¹⁰: is selected from the group consisting of hydroxy and methyl;
- a moiety of formula I-3 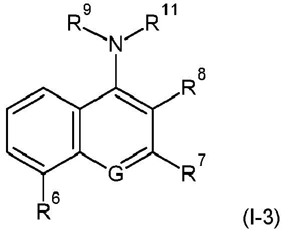 wherein
   - G: is selected from the group consisting of nitrogen and C-H;
   - R⁶: is selected from the group consisting of hydrogen and amino,
   - R⁷ and R⁸: are independently of each other selected from the group consisting of hydrogen, amino and methyl, or together with the carbon atoms to which they are bonded, form a saturated or unsaturated five or six-membered ring,
   - R⁹: is selected from the group consisting of hydrogen, phenyl, saturated branched or unbranched alkyl having 1 to 4 carbon atoms and saturated branched or unbranched aminoalkyl having 1 to 4 carbon atoms and
   - R¹¹: is a bond or hydrogen
   wherein preferably at least one of the groups G, R⁶, R⁷, R⁸, R⁹ or N-R¹¹ provides a heteroatom, preferably selected from oxygen and nitrogen, to which a Photoremovable Protecting Group Moiety B can be covalently bonded;
   and
- a moiety which is derived from a corresponding compound which, upon common chemical modification, provides a respective heteroatom, preferably selected from sulfur, oxygen and nitrogen, to which the Photoremovable Protecting Group Moiety B can be attached, wherein the corresponding compound is selected from the group consisting of 2-mercaptobenzothiazol, dithranol (also known as 1,8,9-trihydroxyanthracene), 3-aminophthalhydrazide (also known as 3-APH), 2,3-dicyanohydroquinone (also known as DCH), 1,1'-binaphthyl-2,2'-diamine (also known as BNDM), 7-aminoquinoline, 2-(4-hydroxyphenylazo)benzoic acid (also known as HABA), 6-aza-2-thiothymine (also known as ATT), p-nitroaniline (also known as PNA), 9-aminoacridine (also known as 9-AA), 2-(2-aminoethylamino)-5-nitropyridine, 1,8-bis(dimethylamino)naphthalene (also known as DMAN), 7-hydroxycoumarin-4-acetic acid, 7,8-dihydroxy-6-methox-ycoumarin (also known as fraxetin), 6,7-dihydroxycoumarin, 7-hydroxycoumarin and 3-hydroxycoumarin (also known as 3-HC).

Where the organic MALDI Matrix Compound Moiety A of the Photo-caged MALDI Matrix Compound is a moiety of formula I-1, it is preferably attached via the (free) bond of the group "X" to the Photoremovable Protecting Group Moiety B to form a Photo-caged MALDI Matrix Compound. A moiety of formula I-1 is preferably derived from a corresponding compound which, upon common chemical modification, provides a group "X" to which the Photoremovable Protecting Group Moiety B can be attached. Preferred corresponding compounds from which moieties of formula I-1 are preferably derived are selected from the group consisting of 2,5-dihydroxybenzoic acid (also known as DHB or gentisic acid), 2,5-dihydroxyacetophenone, 2,6-dihydroxyacetophenone, 2,4,6-trihydroxyacetophenone, 2-(methylamino)benzoic acid (also known as COOH-NHMe) and 2,5-dihydroxyterephthalic acid.

Where the organic MALDI Matrix Compound Moiety A of the Photo-caged MALDI Matrix Compound is a moiety of formula I-2, it is preferably attached via the (free) bond of the oxygen atom attached to the phenyl ring to the Photoremovable Protecting Group Moiety B, to form a Photo-caged MALDI Matrix Compound. A moiety of formula I-2 is preferably derived from a corresponding compound which, upon common chemical modification, provides a respective oxygen atom to which the Photoremovable Protecting Group Moiety B can be attached. Preferred corresponding compounds from which moieties of formula I-2 are preferably derived are selected from the group consisting of alpha-cyano-4-hydroxycinnamic acid (also known as HCCA or CHCA), 4-chloro-alpha-cyanocinnamic acid, sinapinic acid (also known as trans-3,5-dimethoxy-4-hydroxy cinnamic acid or SA), ferulic acid (also known as trans-3-methoxy-4-hydroxy cinnamic acid or FA) and (E)-4-(2,5-dihydroxyphenyl) but-3-en-2-one (also known as 2,5-cDHA).

Where the organic MALDI Matrix Compound Moiety A of the Photo-caged MALDI Matrix Compound is a moiety of formula I-3, it is preferably attached via a (free) bond of a nitrogen atom present in the moiety of formula I-3 to the Photoremovable Protecting Group Moiety B, to form a Photo-caged MALDI Matrix Compound. A moiety of formula I-3 is preferably derived from a corresponding compound, which, upon common chemical modification, provides a respective nitrogen atom to which the Photoremovable Protecting Group Moiety B can be attached. Preferred corresponding compounds from which moieties of formula I-3 are preferably derived are selected from the group consisting of 2-aminoquinoline, 3-aminoquinoline, 4-aminoquinoline, 8-aminoquinoline, N-(1-naphthyl)ethylene diamine dihydrochloride (also known as NEDC), 1,5-diaminonaphthalene (also known as 1,5-DAN) and N-phenyl-2-naphthylamine (also known as PNA).

In a further specific variant of the present invention is preferred a Photo-sensitive MALDI Matrix Composite according to the present invention as defined herein (or a Photo-sensitive MALDI Matrix Composite according to the present invention as described herein as preferred), wherein the Photoremovable Protecting Group Moiety B of the Photo-caged MALDI Matrix Compound is a nitrophenyl group, preferably defined by formula II 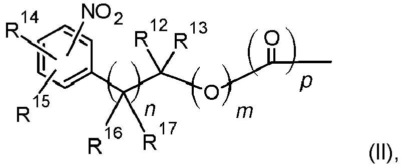 wherein
- R¹²: is selected from the group consisting of hydrogen and methyl;
- R¹³: is selected from the group consisting of hydrogen, methyl and carboxy;
- R¹⁴: is selected from the group consisting of hydrogen, methoxy, carboxy, nitro and -O(CH₂)ₗCOOH, wherein I is an integer in the range from 1 to 3;
- R¹⁵: is selected from the group consisting of hydrogen and methoxy;
- R¹⁶ and R¹⁷: are hydrogen or, together with the carbon atom to which they are bonded, form a carbonyl group; and
- m, n and p: are independently of each other 0 or 1;
and wherein preferably the MALDI Matrix Compound Moiety A is a moiety of formula I-1 as defined above.

Preferred Photoremovable Protecting Group Moieties B of formula II for the purposes of the present invention are selected from the group consisting of 4,5-dimethoxy-2-nitrobenzyl (also known as DMNB), alpha-carboxy-2-nitrobenzyl (also known as CNB), 1-(2-nitrophenyl)ethyl (also known as NPE), 5-carboxymethoxy-2-nitrobenzyl (also known as CMNB), 1-(4,5-dimethoxy-2-nitrophenyl)ethyl (also known as DMNPE) and 2-nitroveratryloxycarbonyl (also known as NVOC).

A Photoremovable Protecting Group Moiety B of formula II is preferably attached to an organic MALDI Matrix Compound Moiety A via the (free) bond next to (and on the right side of) the letter "p" shown in formula II above.

It has been found in own experiments that a nitrophenyl group, preferably a nitrophenyl group of formula II as defined above, is particularly suited as a Photoremovable Protecting Group Moiety B of a Photo-caged MALDI Matrix Compound for use in a Photo-sensitive MALDI Matrix Composite according to the present invention. Photo-caged MALDI Matrix Compounds comprising a nitrophenyl group of formula II as a Photoremovable Protecting Group Moiety B were found to show (i) good or very good solubility in common organic solvents or mixtures of common organic solvents with water, (ii) good or very good formation of crystals, (iii) good or very good stability against evaporation when exposed to a vacuum (in reduced pressure ranges as are common in MALDI-MS methods) and (iv) indication of cleavage and release of the corresponding MALDI matrix compound upon irradiation with radiation of a wavelength in the range of from ≥ 100 nm to ≤ 15 µm, preferably in the range of from ≥ 150 nm to ≤ 750 nm.

It has further been found in own experiments that a Photo-caged MALDI Matrix Compound for use in a Photo-sensitive MALDI Matrix Composite according to the present invention is preferred, wherein a moiety of formula I-1 (as defined above) forms the MALDI Matrix Compound Moiety A and a nitrophenyl group of formula II (as defined above) forms the Photoremovable Protecting Group Moiety B. For such preferred Photo-caged MALDI Matrix Compounds, the above-stated beneficial properties (i) to (iv) were found to be particularly pronounced.

Preferred is therefore a Photo-sensitive MALDI Matrix Composite according to the present invention as defined herein (or a Photo-sensitive MALDI Matrix Composite according to the present invention as described herein as preferred), wherein the Photo-caged MALDI Matrix Compound comprises as Photoremovable Protecting Group Moiety B a nitrophenyl group of formula II (as defined above) and as organic MALDI Matrix Compound Moiety A a moiety of formula I-1 (as defined above).

In a preferred variant of the present invention, a Photo-sensitive MALDI Matrix Composite according to the present invention as defined herein (or a Photo-sensitive MALDI Matrix Composite according to the present invention as described herein as preferred) is preferred, wherein the Photo-caged MALDI Matrix Compound is selected from the group consisting of 1-(5-((4,5-dimethoxy-2-nitrobenzyl)oxy)-2-hydroxyphenyl)ethan-1-one, N¹-(4,5-dimethoxy-2-nitrobenzyl)naphthalene-1,5-diamine, 3-((4,5-dimethoxy-2-nitrobenzyl)oxy)-2*H*-chromen-2-one, 1-(2-((4,5-dimethoxy-2-nitrobenzyl)oxy)-6-hydroxyphenyl)ethan-1-one, 2-((4,5-dimethoxy-2-nitrobenzyl)thio)benzo[d]thiazole, 1-((4,5-dimethoxy-2-nitrobenzyl)oxy)-8-hydroxyanthracen-9(10*H*)-one, 4-((3-acetyl-4-hydroxyphenoxy)methyl)-3-nitrobenzoic acid, 4-(4-(1-(3-acetyl-4-hydroxyphenoxy)ethyl)-2-methoxy-5-nitrophenoxy)butanoic acid, 1-(2-((4,5-dimethoxy-2-nitrobenzyl)oxy)-6-hydroxyphenyl)ethan-1-one, 3-acetyl-4-hydroxyphenyl (4,5-dimethoxy-2-nitrobenzyl) carbonate and mixtures thereof. Accordingly, the Photo-caged MALDI Matrix Compound as listed here before represent preferred Photo-caged MALDI Matrix Compounds according to the present invention.

The present invention also pertains to the use of a Photo-caged MALDI Matrix Compound as described herein (or a Photo-caged MALDI Matrix Compound as described herein as preferred)
- as matrix compound (and/or as MALDI matrix compound) in a method of matrix-assisted laser desorption/ionization mass spectrometry
   and/or
- for preparing a Photo-sensitive MALDI Matrix Composite according to the present invention as defined herein (or a Photo-sensitive MALDI Matrix Composite according to the present invention as described herein as preferred).

Generally, all aspects of the present invention discussed herein in the context of the Photosensitive MALDI Matrix Composite according to the present invention as described herein apply *mutatis mutandis* to the use of a Photo-caged MALDI Matrix Compound according to the present invention as described herein, and *vice versa.*

The use of the Photo-caged MALDI Matrix Compound according to the present invention as defined herein comprises the use as matrix compound in a method of matrix-assisted laser desorption/ionization mass spectrometry where the Photo-caged MALDI Matrix Compound, without further modification as to its chemical structure, assumes the role of a MALDI matrix compound as is known in the art. Preferred is, however, the use of the Photo-caged MALDI Matrix Compound according to the present invention as defined herein, wherein the Photo-caged MALDI Matrix Compound (present as matrix compound or MALDI matrix compound of a Photo-sensitive MALDI Matrix Composite according to the present invention, as defined above) is first irradiated with a first radiation of a wavelength in the range of from ≥ 100 nm to ≤ 15 µm to release a MALDI matrix compound (as described in more detail below), and the so released MALDI matrix compound subsequently assumes the role of a MALDI matrix compound as is known in the art.

For the use of the Photo-caged MALDI Matrix Compound according to the present invention for preparing a Photo-sensitive MALDI Matrix Composite, the Photo-sensitive MALDI Matrix Composite can be prepared from a Photo-caged MALDI Matrix Compound, one or more than one analyte to be analyzed (as defined above) and optionally further components (e.g. including the MALDI matrix compound which can be obtained by photo-cleaving the Photo-caged MALDI Matrix Compound used) by methods known in the art.

More specifically is preferred the use of a Photo-caged MALDI Matrix Compound according to the present invention as described herein (or the use of a Photo-caged MALDI Matrix Compound according to the present invention as described herein as preferred), wherein ≤ 20 % of the total mass of a sample of the Photo-caged MALDI Matrix Compound evaporates after exposure of the sample to a vacuum of ≤ 3300 Pa, preferably of ≤ 10 Pa, for a time period in the range of from ≥ 36 hrs to ≤ 72 hrs, preferably of from ≥ 48 hrs to ≤ 96 hrs, more preferably of from ≥ 72 hrs to ≤ 120 hrs.

As explained above, stability of a MALDI matrix compound, *viz.* a Photo-caged MALDI Matrix Compound, against undesired premature evaporation is of particular value where a vacuum atmosphere needs to be applied for extended periods in MALDI-MS or MALDI-MSI methods.

The present invention further pertains to a matrix-assisted laser desorption/ionization mass spectrometry method, comprising the following steps:
S1) providing or preparing a Photo-sensitive MALDI Matrix Composite according to the present invention as described herein (or a Photo-sensitive MALDI Matrix Composite according to the present invention as described herein as preferred);
S2) irradiating the Photo-sensitive MALDI Matrix Composite as provided or prepared in step S1) with a first radiation of a wavelength suitable to release a MALDI matrix compound from the Photo-caged MALDI Matrix Compound present in the Photosensitive MALDI Matrix Composite,
   wherein the released MALDI matrix compound has a lower molecular mass and/or (preferably "and") a higher vapor pressure at 25 °C than the Photo-caged MALDI Matrix Compound from which it was released,
   to receive a MALDI matrix composite, preferably comprising a MALDI matrix compound and, preferably embedded therein, one or more than one analyte to be analyzed;
S3) irradiating the MALDI matrix composite received in step S2) with a second radiation of a nature and wavelength suitable to result in desorption/ionization of the one or more than one analyte from step S2), and preferably also suitable to result in desorption/ionization of the MALDI matrix compound received in step S2),
   and
S4) providing the one or more than one desorbed and/or ionized analyte from step S3), and preferably the desorbed and/or ionized MALDI matrix compound from step S3), to a mass spectrometer, preferably to the inlet of a mass spectrometer,
   wherein preferably at least the one or one of the more than one analyte is present in the gas phase.

Generally, all aspects of the present invention discussed herein in the context of the Photosensitive MALDI Matrix Composite according to the present invention as described herein and/or to the use of a Photo-caged MALDI Matrix Compound according to the present invention as described herein apply *mutatis mutandis* to the matrix-assisted laser desorption/ionization mass spectrometry method according to the present invention as described herein, and *vice versa.*

As explained above, the Photo-sensitive MALDI Matrix Composite in step S1) can preferably be prepared from a Photo-caged MALDI Matrix Compound (as described above), one or more than one analyte to be analyzed (as described above) and optionally further components (e.g. including the MALDI matrix compound which can be obtained by photo-cleaving the Photo-caged MALDI Matrix Compound used) by methods known in the art.

In step S2), irradiating the Photo-sensitive MALDI Matrix Composite with a first radiation of a suitable wavelength (as defined above or below) preferably has the effect that the Photo-caged MALDI Matrix Compound comprised by the Photo-sensitive MALDI Matrix Composite, which is likewise exposed to said first radiation, is photo-cleaved by the impact of the first radiation, preferably at the site of the covalent bonding between the Photoremovable Protecting Group Moiety B and the organic MALDI Matrix Compound Moiety A, so that a MALDI matrix compound is released. The MALDI matrix compound so released preferably corresponds to the compound from which the organic MALDI Matrix Compound Moiety A was derived.

Preferred is a matrix-assisted laser desorption/ionization mass spectrometry method according to the present invention as described herein (or a matrix-assisted laser desorption/ionization mass spectrometry method according to the present invention as described herein as preferred), wherein the wavelength of the first radiation applied in step S2) and/or the wavelength of the second radiation applied in step S3) are independently of each other in the range of from ≥ 100 nm to ≤ 15 µm, preferably of from ≥ 250 nm to ≤ 10 µm,
wherein preferably
- the first radiation applied in step S2) is or comprises radiation from a laser source, preferably from a pulsed laser source, more preferably from a modulated pulsed laser source,
   wherein preferably the wavelength of the radiation is in the range of from ≥ 150 nm to ≤ 700 nm, more preferably of from ≥ 250 nm to ≤ 400 nm;
   and/or
- the second radiation applied in step S3) is or comprises radiation from a laser source, preferably from a pulsed laser source, more preferably from a modulated pulsed laser source,
   wherein preferably the wavelength of the radiation is in the range of from ≥ 150 nm to ≤ 700 nm, preferably of from ≥ 250 nm to ≤ 400 nm;
   and/or
- the first radiation applied in step S2) and the second radiation applied in step S3) both comprise radiation from a pulsed laser source, preferably from a modulated pulsed laser source,
   wherein preferably the wavelength of the radiation is in each case in the range of from ≥ 150 nm to ≤ 700 nm, preferably of from ≥ 250 nm to ≤ 400 nm.

In a preferred variant of the matrix-assisted laser desorption/ionization mass spectrometry method according to the present invention as described herein, the first radiation applied in step S2) and/or the second radiation applied in step S3) comprise in each case radiation from a pulsed laser source, preferably from a modulated pulsed laser source, wherein the wavelength of the radiation in each case comprises a wavelength selected from the group consisting of 266 nm, 337 nm, 355 nm, 2.94 µm and 10.6 µm, preferably selected from the group consisting of 266 nm, 337 nm and 355 nm.

Also preferred is a matrix-assisted laser desorption/ionization mass spectrometry method according to the present invention as described herein (or a matrix-assisted laser desorption/ionization mass spectrometry method according to the present invention as described herein as preferred), wherein the first radiation applied in step S2) and the second radiation applied in step S3) are of equal nature and wavelength,
wherein preferably
- the irradiation in step S2) and the irradiation in step S3) are performed with the same radiation,
   and/or
- the first radiation applied in step S2) and the second radiation applied in step S3) are the same;
   and/or
- step S2) and step S3) are performed in one single step.

In the preferred variant of the matrix-assisted laser desorption/ionization mass spectrometry method as described here above, release of the MALDI matrix compound from the Photo-caged MALDI Matrix Compound present in the Photo-sensitive MALDI Matrix Composite in step S2) and desorption/ionization of the analyte (and preferably the desorption/ionization of the MALDI matrix compound) in step S3) are accomplished, preferably in one single step, by irradiating the Photo-sensitive MALDI Matrix Composite with radiation of a wavelength in a defined range (preferably with a wavelength in a preferred range as defined above) or with radiation of a preferred wavelength as defined above. In this preferred variant, the release of the MALDI matrix compound and the subsequent desorption/ionization of the analyte (and preferably the desorption/ionization of the MALDI matrix compound) both occur within an extremely short time interval. In this preferred variant, it is therefore not required to prepare and perform two different working steps which might otherwise require different machine settings and/or different sample preparation steps. This preferred variant of the matrix-assisted laser desorption/ionization mass spectrometry method as described here above is therefore a particularly beneficial and efficient variant of the method of the present invention.

Preferred is furthermore a matrix-assisted laser desorption/ionization mass spectrometry method according to the present invention as described herein (or a matrix-assisted laser desorption/ionization mass spectrometry method according to the present invention as described herein as preferred), wherein step S3) comprises irradiating the one or more than one desorbed but not yet ionized analyte, and preferably the desorbed MALDI matrix compound, with a third radiation from a laser source, preferably from a pulsed laser source, more preferably from a modulated pulsed laser source,
wherein preferably
- the wavelength of the third radiation is in the range of from ≥ 150 nm to ≤ 700 nm, preferably of from ≥ 250 nm to ≤ 400 nm
   and/or
- the third radiation is applied to the one or more than one desorbed but not yet ionized analyte when said analyte is in the gas phase, and preferably to the desorbed and/or ionized MALDI matrix compound when said MALDI matrix compound is in the gas phase.

In the preferred variant of the matrix-assisted laser desorption/ionization mass spectrometry method according to the present invention as defined here above, the third radiation, which is combined with a common method of matrix-assisted laser desorption/ionization mass spectrometry, preferably serves for laser-induced postionization of the analyte and/or the MALDI matrix compound to enhance mass spectrometry imaging of certain classes of biomolecules. This method is known under the name "MALDI-2" and is e.g. described by J. Soltwisch et al. in SCIENCE, Vol. 384, Issue 6231 (2015), pp. 211-215 or by J. Soltwisch et al. in Anal. Chem. 92, 13 (2020) pp. 8697-8703.

Preferred is in addition a matrix-assisted laser desorption/ionization mass spectrometry method according to the present invention as described herein (or a matrix-assisted laser desorption/ionization mass spectrometry method according to the present invention as described herein as preferred), wherein a vacuum is applied or maintained in steps S2), S3) and/or S4) (preferably "and"), wherein preferably
- the vacuum is applied or maintained for a time period of from ≥ 36 hrs to ≤ 72 hrs, preferably of from ≥ 48 hrs to ≤ 96 hrs, more preferably of from ≥ 72 hrs to ≤ 120 hrs;
   and/or
- the vacuum comprises a pressure of 3300 Pa or below, more preferably of 5 Pa or below and even more preferably of 0.1 Pa or below.

As explained above, stability of a MALDI matrix compound, *viz.* a Photo-caged MALDI Matrix Compound, against undesired premature evaporation is of particular value where a vacuum atmosphere needs to be applied in MALDI-MS or MALDI-MSI methods. A Photo-caged MALDI Matrix Compound as described herein has a higher molecular mass and a lower vapor pressure (at 23 °C) than the MALDI matrix compound which can be released from it by photo-cleavage. According to the present invention, a MALDI matrix compound can therefore be stabilized against evaporation when exposed to a vacuum by converting it into a corresponding Photo-caged MALDI Matrix Compound. By this method according to the present invention, MALDI matrix compounds with a very low vapor pressure may be made available to MALDI-MS methods for the first time, or MALDI matrix compounds with a low vapor pressure can be made available to MALDI-MSI methods, where stability against evaporation upon exposure to a vacuum for extended time periods is critical, for the first time. In a preferred variant of the a matrix-assisted laser desorption/ionization mass spectrometry method according to the present invention as defined here above, a Photo-caged MALDI Matrix Compound is stabilized against evaporation upon exposure to a vacuum (preferably a vacuum as defined here above) for a time period of from ≥ 4 hrs to ≤ 144 hrs, preferably of from ≥ 12 hrs to ≤ 144 hrs, more preferably of from ≥ 12 hrs to ≤ 72 hrs.

Further preferred is a matrix-assisted laser desorption/ionization mass spectrometry method according to the present invention as described herein (or a matrix-assisted laser desorption/ionization mass spectrometry method according to the present invention as described herein as preferred), wherein step S1) comprises a step of co-crystallizing a Photo-caged MALDI Matrix Compound as defined herein (or a Photo-caged MALDI Matrix Compound as described herein as preferred), with the one or at least one of the more than one analyte as defined herein, preferably to form a Photo-sensitive MALDI Matrix Composite according to the present invention as described herein. Preferably, in this preferred variant of the a matrix-assisted laser desorption/ionization mass spectrometry method according to the present invention as defined herein the Photo-sensitive MALDI Matrix Composite and/or the Photo-caged MALDI Matrix Compound comprised by the Photo-sensitive MALDI Matrix Composite is in each case present in the solid state under the conditions applied in said method, more preferably herein the Photo-sensitive MALDI Matrix Composite and/or the Photo-caged MALDI Matrix Compound comprised by the Photo-sensitive MALDI Matrix Composite is in each case present in the solid state at 23 °C and 101,3 kPa.

Moreover, the present invention pertains to a Photo-caged MALDI Matrix Compound as defined above (or a Photo-caged MALDI Matrix Compound as defined herein as preferred).

Generally, all aspects of the present invention discussed herein in the context of the Photosensitive MALDI Matrix Composite according to the present invention as described herein and/or to the use of a Photo-caged MALDI Matrix Compound according to the present invention as described herein and/or the matrix-assisted laser desorption/ionization mass spectrometry method according to the present invention as described herein apply *mutatis mutandis* to the Photo-caged MALDI Matrix Compound according to the present invention as described herein, and *vice versa.*

### Figures:

The invention is further explained and illustrated by the appended figures, as explained here below:
- Fig. 1:: Fig. 1 shows on the left side crystals of the Photo-caged MALDI Matrix Compound of Ex. 1.1 formed by a spray method (cf. Example 3 below for details) on the surface of an ITO object slide analyzed by optical scanning in 20-fold magnification. Fig. 1 shows on the right side crystals of the Photo-caged MALDI Matrix Compound of Ex. 1.1 formed by a spray method (cf. Example 3 below for details) on the surface of a sliced sample of hog's brain analyzed by optical scanning in 20-fold magnification
- Fig. 2:: Fig. 2 shows crystals of the Photo-caged MALDI Matrix Compound of Ex. 1.1 formed by a spray method (see above) on the surface of an ITO object slide analyzed by scanning electron microscopy in 5000-fold magnification.
- Fig. 3:: Fig. 3 shows crystals of the Photo-caged MALDI Matrix Compound of Ex. 1.1 formed by a spray method (see above) on the surface of an ITO object slide analyzed by scanning electron microscopy in 10000-fold magnification.
- Fig. 4:: Fig. 4 shows crystals of the Photo-caged MALDI Matrix Compound of Ex. 1.1 formed by a spray method (see above) on the surface of a sliced sample of hog's brain analyzed by scanning electron microscopy in 5000-fold magnification.
- Fig. 5:: Fig. 5 shows crystals of the Photo-caged MALDI Matrix Compound of Ex. 1.1 formed by a spray method (see above) on the surface of a sliced sample of hog's brain analyzed by scanning electron microscopy in 10000-fold magnification.
- Fig. 6.1:: Fig. 6.1 shows a bright field scan of a sample of hog's brain (slice) on an object slide of glass, covered with Photo-caged MALDI Matrix Compound of Ex. 1.2 (as described below).
- Fig. 6.2 to Fig. 6.9:: Fig. 6.2 to Fig. 6.9 show MALDI-MSI ion images of the sample of hog's brain from Fig. 6.1 at 50 µm spatial resolution in negative ion mode for different mass-to-charge ratios (Fig. 6.2: m/z = 726.6; Fig. 6.3: m/z = 728.6; Fig. 6.4: m/z = 750.6; Fig. 6.5: m/z = 766.6; Fig. 6.6: m/z = 788.6; Fig. 6.7: m/z = 885.6; Fig. 6.8: m/z = 888.7; Fig. 6.9: m/z = 904.7).
- Fig. 7.1: Fig. 7.1: shows a bright field scan of a sample of hog's brain (slice) on an object slide of glass, covered with Photo-caged MALDI Matrix Compound of Ex. 1.1 (as described below).
- Fig. 7.2 to Fig. 7.9:: Fig. 7.2 to Fig. 7.9 show MALDI-MSI ion images of the sample of hog's brain from Fig. 7.1 at 50 µm spatial resolution in negative ion mode for different mass-to-charge ratios (Fig. 7.2: m/z = 726.6; Fig. 7.3: m/z = 728.6; Fig. 7.4: m/z = 750.6; Fig. 7.5: m/z = 766.6; Fig. 7.6: m/z = 788.6; Fig. 7.7: m/z = 885.6; Fig. 7.8: m/z = 888.7; Fig. 7.9: m/z = 904.7).
- Fig. 8.1: Fig. 8.1: shows a bright field scan of a sample of hog's brain (slice) on an object slide of glass, covered with Photo-caged MALDI Matrix Compound of Ex. 1.5 (as described below).
- Fig. 8.2 to Fig. 8.9:: Fig. 8.2 to Fig. 8.9 show MALDI-MSI ion images of the sample of hog's brain from Fig. 8.1 at 50 µm spatial resolution in negative ion mode for different mass-to-charge ratios (Fig. 8.2: m/z = 726.6; Fig. 8.3: m/z = 728.6; Fig. 8.4: m/z = 750.6; Fig. 8.5: m/z = 766.6; Fig. 8.6: m/z = 788.6; Fig. 8.7: m/z = 885.6; Fig. 8.8: m/z = 888.7; Fig. 8.9: m/z = 904.7).
- Fig. 9:: Fig. 9 shows a MALDI-TOF MS spectrum of the Photo-caged MALDI Matrix Compound of Ex. 1.2 (DMNB-1,5-DAN) in negative ion mode.
- Fig. 10:: Fig. 10 shows a MALDI-TOF MS spectrum of the Photo-caged MALDI Matrix Compound of Ex. 1.1 (DMNB-2,5-DHAP) in negative ion mode.
- Fig. 11:: Fig. 11 shows a MALDI-TOF MS spectrum of the Photo-caged MALDI Matrix Compound of Ex. 1.5 (DMNB-2MBT) in negative ion mode.

### Examples:

The following examples are meant to further explain and illustrate the present invention without limiting its scope.

### Example 1: Synthesis of Photo-caged MALDI Matrix Compounds according to the invention

### Ex. 1.1: 1-(5-((4,5-Dimethoxy-2-nitrobenzyl)oxy)-2-hydroxyphenyl)ethan-1-one (DMNB-2,5-DHAP)

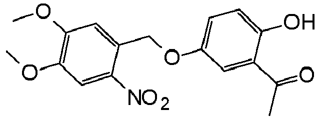

1-(Bromomethyl)-4,5-dimethoxy-2-nitrobenzene (2,5 g, 9,05 mmol, 1 eq), 1-(2,5 dihydroxyphenyl)ethan-1-one (1,38 g, 9,05 mmol, 1 eq) and potassium carbonate (2,5g, 18,11 mmol, 2 eq) were suspended in 50 ml of acetone. The mixture was stirred under reflux for 3h after which it was filtered while hot. The filtrate was concentrated under reduced pressure to afford a crude brown solid which was purified via reversed-phase high performance liquid chromatography (RP-HPLC) to afford the desired product (see above, 1,06 g, 4,49 mmol) as a pale yellow solid in 50% yield. ¹H-NMR (600 MHz, Chloroform-d) δ [ppm] 11.94 (d, J = 2.3 Hz, 1H), 7.78 (d, J = 2.2 Hz, 1H), 7.35 (d, J = 2.2 Hz, 1H), 7.32 (d, J = 2.8 Hz, 1H), 7.23 (dt, J = 9.2, 2.8 Hz, 1H), 6.97 (dd, J = 8.9, 2.3 Hz, 1H), 5.47 (d, J = 2.2 Hz, 2H), 4.00 (dd, J = 9.9, 2.3 Hz, 6H), 2.64 (d, J = 2.3 Hz, 3H). ¹³C-NMR (151 MHz, Chloroform-d) δ 203.94, 157.41, 153.96, 150.20, 148.03, 139.18, 128.85, 125.23, 119.55, 119.35, 115.33, 109.48, 108.07, 68.28, 56.50, 56.43, 26.80. MS [M⁺Na]⁺ 370.14.

The Photo-caged MALDI Matrix Compound of Example 1.1 (Ex. 1.1) can be regarded as being prepared from the MALDI matrix compound 2,5-dihydroxyacetophenone (to form the MALDI Matrix Compound Moiety A) and the compound 1-(bromomethyl)-4,5-dimethoxy-2-nitrobenzene, serving to attach the photoremovable protecting group 4,5-dimethoxy2-nitrobenzyl to the MALDI matrix compound (to form the Photoremovable Protecting Group Moiety B). The Photoremovable Protecting Group Moiety B has the molecular formula C₉H₁₀O₄N (molar mass 196.1 g/mol) and is covalently bonded to an oxygen atom of the MALDI Matrix Compound Moiety A. The MALDI Matrix Compound Moiety A has the molecular formula C₈H₇O₃ and a molar mass of 151.1 g/mol.

### Ex. 1.2: N¹-(4,5-dimethoxy-2-nitrobenzyl)naphthalene-1,5-diamine (DMNB-1,5-DAN)

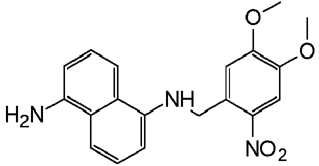

A mixture of 1-(bromomethyl)-4,5-dimethoxy-2-nitrobenzene (1 g, 3,62 mmol, 1 eq), naphthalene-1,5-diamine (1,15 g, 7,24 mmol, 2 eq) and cesium carbonate (1,18 g, 3,62 mmol, 1 eq) were suspended in 30 ml of DMF 30 ml. The mixture was stirred and heated to 60 °C for 5 h after which it was allowed to cool down to room temperature and it was concentrated to constant weight under reduced pressure. The solid residue was purified via RP-HPLC affording the desired product (see above, 0,22g, 0,62 mmol) as a pale brown solid in 18% yield. ¹H-NMR (600 MHz, Chloroform-d) δ 7.78 (s, 1H), 7.37 (dt, J = 8.6, 0.9 Hz, 1H), 7.32 (dd, J = 8.5, 7.2 Hz, 1H), 7.28 - 7.19 (m, 2H), 7.16 (s, 1H), 6.83 (dd, J = 7.2, 1.0 Hz, 1H), 6.45 (dd, J = 7.0, 1.5 Hz, 1H), 5.32 (s, 2H), 4.95 (s, 2H), 3.97 (s, 3H), 3.73 (s, 3H). ¹³C-NMR (151 MHz, Chloroform-d) δ 153.72, 147.67, 142.99, 142.85, 140.18, 130.88, 125.48, 125.41, 124.24, 124.22, 110.84, 110.71, 110.40, 109.98, 108.47, 105.45, 56.40, 56.32, 46.56. MS [M⁺Na]⁺ 376.18.

### Ex. 1.3: 3-((4,5-Dimethoxy-2-nitrobenzyl)oxy)-2H-chromen-2-one

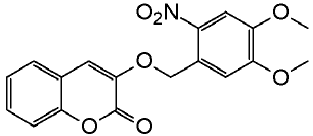

3-Hydroxy-2H-chromen-2-one (0,59 g, 3,62 mmol, 1 eq) and 1-(bromomethyl)-4,5-dimethoxy-2-nitrobenzene (1 g, 3,63 mmol, 1 eq) were dissolved in 50 ml of acetone followed by the addition of potassium carbonate (1,18 g, 3,63 mmol, 1 eq). The mixture was refluxed for 5 h after which volatiles were removed under reduced pressure and the residue was redissolved in dichloromethane and washed with 1M acq. NaOH solution (3 x 25 ml). The organic layer was dried over anhydrous Na₂SO₄ filtered and concentrated under reduced pressure to afford 1,2 g of a beige solid. The solid was purified via normal phase high performance liquid chromatography (NP-HPLC) affording the desired product (see above, 0,815 g, 2,28 mmol) as a beige solid in 63% yield. ¹H-NMR (600 MHz, Chloroform-d) δ 7.79 (s, 1H), 7.54 (s, 1H), 7.46 (dd, J = 7.7, 1.5 Hz, 1H), 7.43 (ddd, J = 8.7, 7.4, 1.6 Hz, 1H), 7.34 (dd, J = 8.3, 1.1 Hz, 1H), 7.29 (td, J = 7.5, 1.2 Hz, 1H), 7.05 (s, 1H), 5.71 - 5.39 (m, 2H), 4.02 (d, J = 35.5 Hz, 6H). ¹³C-NMR (151 MHz, Chloroform-d) δ 157.36,154.34,149.79, 148.18, 143.03, 138.78, 128.97, 127.54, 126.78, 124.86, 119.35, 116.34, 115.26, 109.34, 107.96, 67.92, 56.67, 56.43. MS [M⁺Na]⁺ 380.32.

### Ex. 1.4: 1-(2-((4,5-Dimethoxy-2-nitrobenzyl)oxy)-6-hydroxyphenyl)ethan-1-one

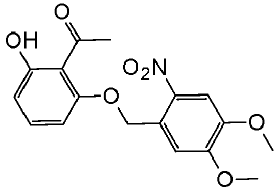

1-(Bromomethyl)-4,5-dimethoxy-2-nitrobenzene (1 g, 3,62 mmol, 1 eq), 1-(2,6-dihydroxyphenyl)ethan-1-one (1,1 g, 7,24 mmol, 1 eq) and potassium carbonate (1 g, 7,24 mmol, 2 eq) were suspended in 25 ml of acetone. The mixture was stirred under reflux for 3h after which it was filtered while hot. The filtrate was concentrated under reduced pressure to afford a crude brown solid which was purified via RP-HPLC to afford the desired product (see above, 0,51 g, 1,47 mmol) as a pale yellow solid in 40% yield. ¹H-NMR (600 MHz, Chloroform-d) δ 13.14 (s, 1H), 7.80 (s, 1H), 7.35 (t, J = 8.3 Hz, 1H), 7.12 (s, 1H), 6.65 (dd, J = 8.4, 1.0 Hz, 1H), 6.41 (dd, J = 8.4, 1.0 Hz, 1H), 5.62 (s, 2H), 4.01 (s, 3H), 3.93 (s, 3H), 2.73 (s, 3H). ¹³C-NMR (151 MHz, Chloroform-d) δ 204.51, 164.67, 159.99, 153.79, 148.42, 139.69, 136.17, 127.38, 111.78, 111.64, 110.13, 108.36, 102.89, 68.56, 56.51, 56.48, 33.79.MS [M+Na]+ 370.33.

### Ex. 1.5: 2-((4,5-Dimethoxy-2-nitrobenzyl)thio)benzo[d]thiazole (DMNB-2MBT)

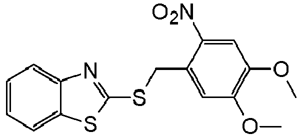

A mixture of benzo[d]thiazole-2(3*H*)-thione (0,48 g, 2,89 mmol, 0,8 eq), 1-(bromomethyl)-4,5-dimethoxy-2-nitrobenzene (1 g, 3,62 mmol, 1 eq) and triethylamine (0,76 ml, 5,43 mmol, 1,5 eq) were stirred in 30 ml of acetonitrile at room temperature for 18 h. Volatiles were removed under reduced pressure and the residue was purified via RP-HPLC affording the desired product (see above, 0,2 g 0,55 mmol) as a pale pink solid in 15% yield. ¹H-NMR (600 MHz, Chloroform-d) δ 7.95 - 7.80 (m, 1H), 7.76 (ddd, J = 8.0, 1.2, 0.6 Hz, 1H), 7.72 (s, 1H), 7.49 - 7.38 (m, 2H), 7.32 (ddd, J = 8.2, 7.3, 1.2 Hz, 1H), 4.98 (s, 2H), 3.95 (d, J = 2.4 Hz, 6H). ¹³C-NMR (151 MHz, Chloroform-d) δ 166.43, 153.01, 152.85, 148.27, 140.23, 135.63, 128.44, 126.12, 124.38, 121.21, 121.05, 114.42, 108.32, 56.39, 56.38, 34.92. MS [M⁺Na]⁺ 385.05.

### Ex. 1.6: 1-((4,5-Dimethoxy-2-nitrobenzyl)oxy)-8-hydroxyanthracen-9(10H)-one

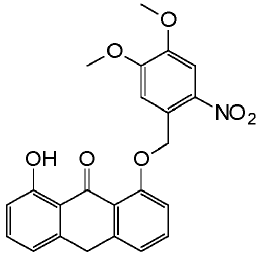

A mixture of 1,8-dihydroxyanthracen-9(10*H*)-one (1,64 g, 7,24 mmol, 1 eq), 1-(bromomethyl)-4,5-dimethoxy-2-nitrobenzene (1 g, 3,62 mmol, 1 eq) and potassium carbonate (1 g, 7,24 mmol, 2 eq) was refluxed in 50 ml of acetone for 4 h. Volatiles were removed and the residue was purified via RP-HPLC affording the desired product (see above, 0,25 g, 0,59 mmol) as a beige solid in 16% yield. ¹H NMR (600 MHz, Chloroform-d) δ 12.15 (s, 2H), 7.69 (s, 1H), 7.41 (dd, J = 8.3, 7.5 Hz, 2H), 6.94 (dd, J = 8.4, 1.0 Hz, 2H), 6.64 (dt, J = 7.4, 0.9 Hz, 2H), 5.67 (s, 1H), 4.58 (t, J = 7.1 Hz, 1H), 3.98 (s, 3H), 3.59 (s, 3H), 3.24 (d, J = 7.2 Hz, 2H). ¹³C NMR (151 MHz, Chloroform-d) δ 193.32, 163.03, 152.07, 147.82, 145.52, 141.23, 136.14, 128.02, 119.81, 116.30, 115.36, 115.11, 108.07, 56.33, 56.17, 48.83, 44.77. MS [M⁺Na]⁺ 444.16.

### Ex. 1.7: 4-((3-Acetyl-4-hydroxyphenoxy)methyl)-3-nitrobenzoic acid

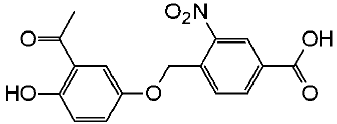

### Ex. 1.7a: Methyl 4-(bromomethyl)-3-nitrobenzoate

4-(Bromomethyl)-3-nitrobenzoicacid (1 g, 3,65 mmol, 1 eq) was dissolved in 10 ml of methanol followed by the addition of concentrated sulfuric acid (400 µl). The mixture was refluxed for 2 h after which it was allowed to reach room temperature and it was concentrated under reduced pressure. The residue was purified via RP-HPLC to afford the desired product of Ex. 1.7a (0,95 g, 3,46 mmol) as a yellowish solid in 95% yield. ¹H-NMR (600 MHz, Chloroform-d) δ 8.68 (d, J = 1.7 Hz, 1H), 8.26 (dd, J = 8.0, 1.8 Hz, 1H), 7.74 - 7.64 (m, 1H), 4.86 (s, 2H), 4.00 (s, 3H), 3.99 (s, 1H), 3.54 (s, 1H). ¹³C-NMR (151 MHz, Chloroform-d) δ 164.45, 137.06, 134.14, 132.85, 131.72, 126.54, 125.83, 52.89, 27.95. MS [M⁺Na]⁺ 297.06.

### Ex. 1.7b: Methyl 4-((3-acetyl-4-hydroxyphenoxy)methyl)-3-nitrobenzoate

Methyl 4-(bromomethyl)-3-nitrobenzoate (0,95 g, 3,46 mmol, 1 eq), 1-(2,5 dihydroxyphenyl)ethan-1-one (0,53 g, 3,46 mmol, 1 eq) and potassium carbonate (0,96 g, 6,93 mmol, 2 eq) were suspended in 20 ml of acetone. The mixture was stirred under reflux for 3h after which it was filtered while hot. The filtrate was concentrated under reduced pressure to afford a crude brown solid which was purified via RP-HPLC to afford the desired product of Ex. 1.7b (0,26 g, 0,75 mmol) as a pale yellow solid in 22% yield. ¹H-NMR (600 MHz, Chloroform-d) δ 11.93 (s, 1H), 8.82 (d, J = 1.7 Hz, 1H), 8.37 (dd, J = 8.2, 1.7 Hz, 1H), 8.05 (dt, J = 8.1, 1.0 Hz, 1H), 7.33 (d, J = 3.0 Hz, 1H), 7.22 (dd, J = 9.0, 3.0 Hz, 1H), 6.98 (d, J = 9.1 Hz, 1H), 5.73 - 5.30 (m, 2H), 4.02 (s, 3H), 2.65 (s, 3H). ¹³C-NMR (151 MHz, Chloroform-d) δ 203.90, 164.73, 157.53, 149.93, 146.88, 138.03, 134.47, 130.92, 128.88, 126.14, 124.88, 119.62, 119.38, 115.43, 67.82, 52.85, 26.81. MS [M⁺Na]⁺ 368.3.

### Ex. 1.7: see above

Methyl 4-((3-acetyl-4-hydroxyphenoxy)methyl)-3-nitrobenzoate (0,26 g, 0,75 mmol, 1 eq) was dissolved in a 1:1 mixture of THF and water. Lithium hydroxide (0,036 g, 1,5 mmol, 2 eq) was added and the mixture was warmed up to 40°C while stirring for 3 h. The mixture was allowed to cool down to room temperature after which trifuoroacetic acid was added until pH 3 followed by purification via RP-HPLC affording the desired product of Ex. 1.7 (see above, 0,15 g, 0,45 mmol) as a pale yellow solid in 60% yield. ¹H-NMR (600 MHz, DMSO-d6) δ 11.46 (s, 1H), 8.54 (d, J = 1.7 Hz, 1H), 8.29 (dd, J = 8.0, 1.8 Hz, 1H), 7.97 (d, J = 8.0 Hz, 1H), 7.46 (t, J = 2.6 Hz, 1H), 7.27 (dd, J = 9.0, 3.1 Hz, 1H), 6.93 (d, J = 9.1 Hz, 1H), 5.53 (s, 2H), 2.63 (s, 3H). ¹³C-NMR (151 MHz, DMSO) δ 203.99, 165.78, 155.81, 150.41, 147.67, 137.75, 134.56, 131.92, 129.99, 125.78, 124.76, 121.02, 119.13, 116.20, 67.66, 28.58. MS [M⁺Na]⁺ 354.06.

### Ex. 1.8: 4-(4-(1-(3-Acetyl-4-hydroxyphenoxy)ethyl)-2-methoxy-5-nitrophenoxy)butanoic acid

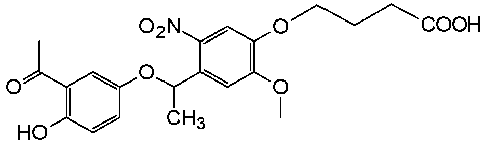

### Ex. 1.8a: Methyl 4-(4-(1-hydroxyethyl)-2-methoxy-5-nitrophenoxy)butanoate

4-(4-(1-hydroxyethyl)-2-methoxy-5-nitrophenoxy)butanoic acid (0,6 g, 2 mmol, 1 eq) was dissolved in 5 ml of MeOH followed by the addition of trimethylsilyl chloride (505µl, 4 mmol, 2 eq). The mixture was stirred at room temperature for 20 minutes. Volatiles were removed under reduced pressure affording the desired product of Ex. 1.8a (0,58 g, 1,84 mmol) as a white solid in 92% yield and sufficiently pure to be used in the next step. ¹H-NMR (600 MHz, Chloroform-d) δ 7.58 (s, 1H), 7.31 (s, 1H), 5.58 (q, J = 6.3 Hz, 1H), 4.13 (tt, J = 6.3, 3.1 Hz, 2H), 3.99 (s, 3H), 3.72 (s, 3H), 2.57 (t, J = 7.2 Hz, 2H), 2.27 - 2.16 (m, 2H), 1.57 (d, J = 6.3 Hz, 3H).¹³C-NMR (151 MHz, Chloroform-d) δ 173.36, 154.13, 146.91, 139.55, 136.90, 109.09, 108.69, 68.23, 65.77, 56.34, 51.73, 30.37, 24.26. MS [M⁺Na]⁺ 336.29.

### Ex. 1.8b: Methyl 4-(4-(1-(3-acetyl-4-hydroxyphenoxy)ethyl)-2-methoxy-5-nitrophenoxy) butanoate

A mixture of 1-(2,5 dihydroxyphenyl)ethan-1-one (0,24 g, 1,55 mmol, 1 eq) and methyl 4-(4-(1-hydroxyethyl)-2-methoxy-5-nitrophenoxy)butanoate (0,51 g, 1,63 mmol, 1,05 eq) was dissolved in 1 ml of THF followed by the addition of diisopropyl azodicarboxylate (321µl, 1,63 mmol, 1,05 eq) and triphenylphosphine (0,43 g, 1,63 mmol, 1,05 eq). The mixture was sonicated for 20 minutes after which the volatiles were removed under reduced pressure and the residue was purified via RP-HPLC affording the desired product of Ex. 1.8b (0,26 g, 0,58 mmol) as a pale brown solid in 37% of yield. ¹H-NMR (600 MHz, DMSO-d6) δ 12.17 (s, 1H), 11.38 (s, 1H), 7.59 (s, 1H), 7.28 (d, J = 3.1 Hz, 1H), 7.22 (s, 1H), 7.13 (dd, J = 9.0, 3.1 Hz, 1H), 6.84 (d, J = 9.0 Hz, 1H), 5.94 (q, J = 6.2 Hz, 1H), 4.06 (td, J = 6.5, 2.4 Hz, 2H), 3.87 (s, 3H), 2.37 (t, J = 7.3 Hz, 2H), 1.94 (h, J = 6.6 Hz, 2H), 1.67 (d, J = 6.2 Hz, 3H). ¹³C-NMR (151 MHz, Chloroform-d) δ 203.89, 173.30, 157.00, 154.47, 149.25, 147.34, 139.83, 134.03, 125.66, 119.44, 119.20, 115.38, 108.86, 108.47, 72.28, 68.19, 56.34, 51.73, 30.31, 26.66, 26.61, 24.22, 23.55. MS [M⁺Na]⁺ 470.14.

### Ex. 1.8: see above

Methyl 4-(4-(1-(3-acetyl-4-hydroxyphenoxy)ethyl)-2-methoxy-5-nitrophenoxy)butanoate (0,27 g, 0,6 mmol, 1 eq) was dissolved in a 1:1 mixture of THF and water. Lithium hydroxide (0,029 g, 1,2 mmol, 2 eq) was added and the mixture was warmed up to 40°C while stirring for 3 h. The mixture was allowed to cool down to room temperature after which trifuoroacetic acid was added until pH 3 followed by purification via RP-HPLC affording the desired product of Ex. 1.8 (see above, 0,22 g, 0,5 mmol) as a pale yellow solid in 85% yield. ¹H-NMR (600 MHz, DMSO-d6) δ 12.17 (s, 1H), 11.38 (s, 1H), 7.59 (s, 1H), 7.28 (d, J = 3.1 Hz, 1H), 7.22 (s, 1H), 7.13 (dd, J = 9.0, 3.1 Hz, 1H), 6.84 (d, J = 9.0 Hz, 1H), 5.94 (q, J = 6.2 Hz, 1H), 4.06 (td, J = 6.5, 2.4 Hz, 2H), 3.87 (s, 3H), 2.37 (t, J = 7.3 Hz, 2H), 1.94 (h, J = 6.6 Hz, 2H), 1.67 (d, J = 6.2 Hz, 3H). ¹³C-NMR (151 MHz, DMSO) δ 203.78, 174.43, 155.63, 154.06, 149.49, 147.35, 140.41, 133.01, 125.68, 120.87, 119.08, 117.44, 109.40, 109.01, 72.46, 68.35, 56.72, 30.39, 28.32, 24.44, 23.39. MS [M⁺Na]⁺ 456.10.

### Ex. 1.9: 1-(2-((4,5-Dimethoxy-2-nitrobenzyl)oxy)-6-hydroxyphenyl)ethan-1-one

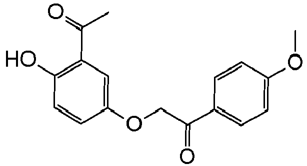

2-Bromo-1-(4-methoxyphenyl)ethan-1-one (1 g, 4,36 mmol, 1 eq), 1-(2,5 dihydroxyphenyl)ethan-1-one (0,66 g, 4,36 mmol, 1 eq) and potassium carbonate (1,2 g, 8,73 mmol, 2 eq) were suspended in 50 ml of acetone. The mixture was stirred overnight at room temperature after which it was filtered. The filtrate was concentrated under reduced pressure to afford a crude brown solid which was purified via RP-HPLC to afford the desired product 9 (0,1 g, 0,33 mmol) as a pale yellow solid in 8% yield. ¹H-NMR (600 MHz, Chloroform-d) δ 11.88 (s, 1H), 8.11 - 7.92 (m, 2H), 7.32 (s, 1H), 7.16 (dd, J = 9.1, 3.0 Hz, 1H), 7.08 - 6.95 (m, 2H), 6.93 (d, J = 9.1 Hz, 1H), 5.22 (s, 2H), 3.91 (s, 3H), 2.60 (s, 3H). ¹³C-NMR (151 MHz, Chloroform-d) δ 204.02, 192.99, 164.19, 157.28, 150.24, 130.48, 127.45, 124.63, 119.32, 119.27, 115.99, 114.10, 71.80, 55.57, 26.78. MS [M⁺Na]⁺ 323.06.

### Ex. 1.10: 3-Acetyl-4-hydroxyphenyl (4,5-dimethoxy-2-nitrobenzyl) carbonate

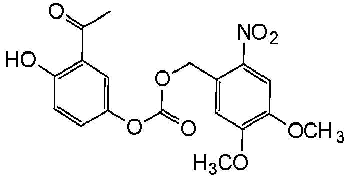

1-(2,5-Dihydroxyphenyl)ethan-1-one (0,14 g, 0,91 mmol, 1 eq) was dissolved in 10 ml of DCM followed by the addition of triethylamine (0,25 ml, 1,82 mmol, 2 eq). The mixture was cooled down to 0 °C after which a solution of 4,5-dimethoxy-2-nitrobenzyl carbonochloridate (0,25 g, 0,91 mmol, 1 eq) in 10 ml of dichloromethane was dropped within 1 h. The mixture was stirred at 0 °C for one additional hour. The volatiles were removed and the solid residue was crystallized from MeOH/H₂O affording the desired product 10 (0,127 g, 0,32 mmol) as a pale yellow solid in 36% yield. ¹H-NMR (600 MHz, Chloroform-d) δ 12.18 (s, 1H), 7.79 (s, 1H), 7.60 (d, J = 2.9 Hz, 1H), 7.34 (dd, J = 9.0, 2.9 Hz, 1H), 7.14 (s, 1H), 7.03 (d, J = 9.0 Hz, 1H), 5.71 (d, J = 0.6 Hz, 2H), 4.02 (d, J = 22.4 Hz, 6H), 2.65 (s, 3H). ¹³C-NMR (151 MHz, Chloroform-d) δ 203.70, 160.33, 153.67, 153.41, 148.67, 142.43, 139.94, 129.47, 125.59, 122.19, 119.53, 119.23, 110.50, 108.35, 67.35, 56.58, 56.48, 26.73. MS [M⁺Na]⁺ 414.05

### Example 2: Solubility tests with Photo-caged MALDI Matrix Compounds according to the invention

Photo-caged MALDI Matrix Compounds according to the invention were prepared as described in Example 1 above and tested for solubility in different solvents (including solvent systems). The solvents (including solvent systems) used in this Example 2 are listed here below in Table 1:

**Table 1: Solvents or solvent systems used for solubility tests**

| **Name** | **Solvent / solvent system** |
|---|---|
| Solvent 1 | Acetonitrile |
| Solvent 2 | Acetonitrile / water (v/v 90/10) |
| Solvent 3 | Acetonitrile / water (v/v 90/10) + 0.1 % trifluoroacetic acid |
| Solvent 4 | Acetonitrile / water (v/v 85/15) + 0.1 % trifluoroacetic acid |
| Solvent 5 | Methanol / water (v/v 90/10) |
| Solvent 6 | Methanol / water (v/v 90/10) + 0.1 % trifluoroacetic acid |
| Solvent 7 | Methanol / water (v/v 90/10) + 50 mM (final conc.) NH₄HCO₃ |
| Solvent 8 | Methanol / water (v/v 85/15) |
| Solvent 9 | Methanol / water (v/v 85/15) + 0.1 % trifluoroacetic acid |
| Solvent 10 | Methanol / water (v/v 85/15) + 50 mM (final conc.) NH₄HCO₃ |

The solubilities found in the solubility tests of this Example 2 are shown in Table 2 below. The numbers of the compounds used in this Example 2 refer to the example numbers of Example 1 above. In Table 2 below, the mark "X" in a cell means, that the respective compound was completely dissolved in the respective solvent or solvent system in a concentration of 2.5 mg/mL. The mark "O" in a cell means, that the respective compound was not completely dissolved at an intended concentration was 2.5 mg/mL in the respective solvent or solvent system.

**Table 2: Results of solubility tests with Photo-caged MALDI Matrix Compounds**

| **Name** | **Ex.** 1.1 | **Ex. 1.2** | **Ex. 1.3** | **Ex. 1.4** | **Ex. 1.5** | **Ex. 1.6** |
|---|---|---|---|---|---|---|
| Solvent 1 | X | X | X | X | X | ○ |
| Solvent 2 | X | X | ○ | X | X | ○ |
| Solvent 3 | X | X | ○ | X | X | ○ |
| Solvent 4 | X | X | ○ | X | X | ○ |
| Solvent 5 | ○ | X | ○ | ○ | X | ○ |
| Solvent 6 | X | X | ○ | ○ | ○ | ○ |
| Solvent 7 | ○ | X | ○ | ○ | ○ | ○ |
| Solvent 8 | ○ | X | ○ | ○ | ○ | ○ |
| Solvent 9 | ○ | X | ○ | ○ | ○ | ○ |
| Solvent 10 | ○ | X | ○ | ○ | ○ | ○ |

From the results shown in Table 2 above it can be seen that the Photo-caged MALDI Matrix Compound according to the invention of Ex. 1.2 showed excellent solubility in the solvents or solvent systems under review and the Photo-caged MALDI Matrix Compounds according to the invention of Ex. 1.1, 1.4 and 1.5 showed very good solubility in the solvents or solvent systems under review.

### Example 3: Crystallisation test with Photo-caged MALDI Matrix Compound according to the invention

The Photo-caged MALDI Matrix Compound of Ex. 1.1 according to the invention was prepared as described in Example 1 above and sprayed tested for its crystallization properties in a spraying method on (i) an indium tin-oxide (ITO) object slide and (ii) on a sliced sample of hog's brain.

In each case, a solution of the Photo-caged MALDI Matrix Compound of Ex. 1.1 (concentration 2.5 mg/mL in acetonitrile/water v/v 4/1) was sprayed onto the surfaces of (i) an ITO object slide and (ii) on a sliced sample of hog's brain with a MALDI HTX M5 Sprayer^{™} (by HTX Technologies, LLC, USA).

The operating parameters applied were as follows: Temperature of spraying nozzle: 50 °C /flow rate: 0.06 mL/min / speed of spraying nozzle: 1000 mm/min / spraying distance: 40 mm.

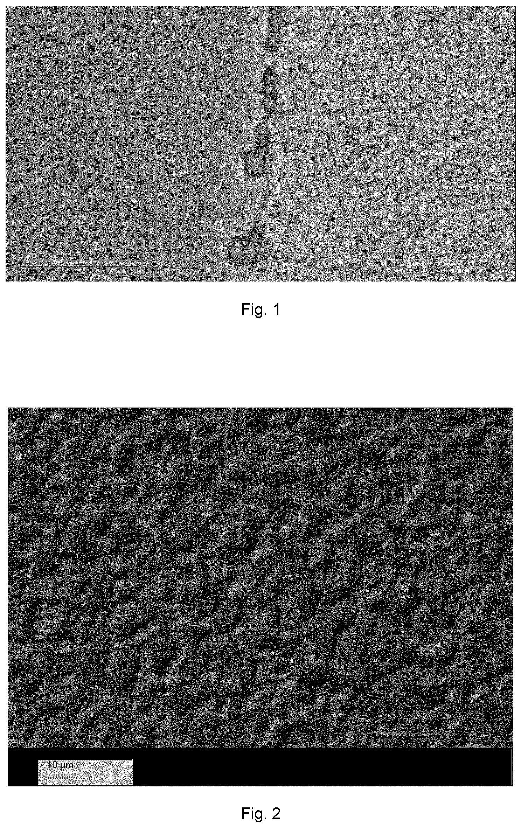

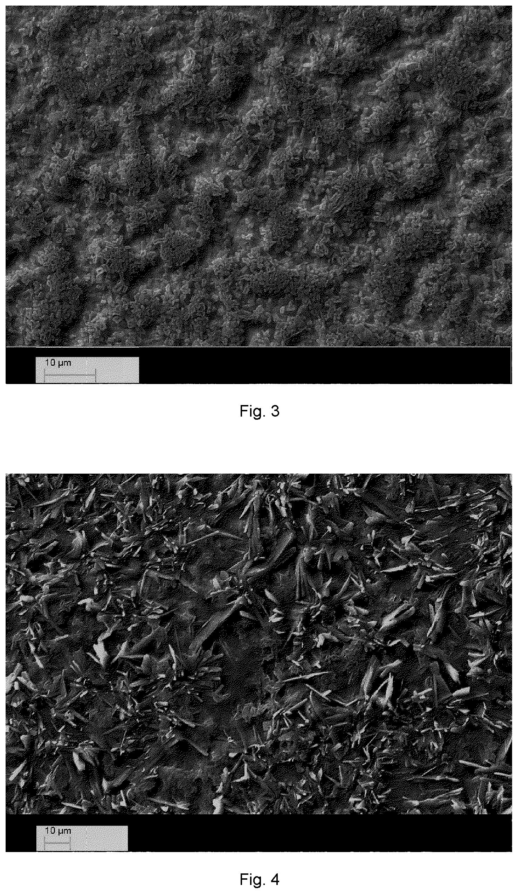

The resulting crystals on the (i) an ITO object slide and (ii) on a sliced sample of hog's brain where then analyzed by optical scanning (in 20-fold magnification) and by scanning electron microscopy (in 5000-fold and 10000-fold magnification), as shown in Fig. 1 to 6.

It was found that the distribution of crystals of the Photo-caged MALDI Matrix Compound of Ex. 1.1 formed by this spray method was even in larger domains while in smaller domains further optimization would be required to improve evenness of the crystal distribution.

The average size of the resulting crystals of the Photo-caged MALDI Matrix Compound of Ex. 1.1 on the ITO object slide was about 1 µm (as determined by analysis of the scanning electron microscopy photographies).

### Example 4: Test for vacuum stability of Photo-caged MALDI Matrix Compound according to the invention

For a determination of stability of the Photo-caged MALDI Matrix Compounds according to the present invention against evaporation upon exposure to a vacuum, the Photo-caged MALDI Matrix Compounds of Examples Ex. 1.1, Ex. 1.2, Ex. 1.3, Ex. 1.4, Ex. 1.5 and Ex. 1.6, respectively, were dissolved in acetonitrile (max. conc. 2.5 mg/mL in each case) and a drop of the resulting solutions (1 µL) was pipetted to an ITO object slide each.

Similarly, for comparison, solutions of the corresponding MALDI matrix compounds (2,5-dihydroxyacetophenone; 1,5-diaminonaphthalene; 3-hydroxycoumarin; 2,6-dihydroxyacetophenone, 2-mercaptobenzothiazole and dithranol, respectively) were dissolved in acetonitrile/water (v/v 1/1; conc. 5 mg/mL in each case) and a drop of the resulting solutions (1 µL) was pipetted to an ITO object slide each, too.

The six object slides carrying the Photo-caged MALDI Matrix Compounds and the six object slides carrying the corresponding MALDI matrix compounds were then transferred to a MALDI TOF spectrometer (Bruker UltrafleXtreme MALDI-TOF MS) and exposed to a vacuum (which was equivalent to usual operating conditions for a MALDI TOF process run) for a time period of 16 h.

Pictures were made by optical scanning of the ITO object slides of all 12 samples at the start of the experiment (t = 0, before exposure to a vacuum, after evaporation of the solvent) and at the end of the experiment (t = 16 h, after 16 hours exposure to a vacuum).

From a comparison of the pictures from optical scanning by visual inspection it was found that no visible losses of the six Photo-caged MALDI Matrix Compound samples could be identified, while considerable losses of all six corresponding MALDI matrix compounds were identified.

It can therefore be concluded that the Photo-caged MALDI Matrix Compounds showed a significantly improved stability against evaporation upon exposure to a vacuum when compared to their respective corresponding MALDI matrix compounds.

### Example 5: Photo-cleavage of Phot-caged MALDI Matrix Compound according to the invention and release of corresponding MALDI matrix compound

For a determination of the ability of the Photo-caged MALDI Matrix Compounds according to the present invention to be cleaved upon exposure to radiation of a wavelength in the range of from ≥ 100 nm to ≤ 15 µm, in particular upon exposure to radiation of a wavelength of 366 nm, the Photo-caged MALDI Matrix Compounds of Examples Ex. 1.1, Ex. 1.2, 1.4 and Ex. 1.5, respectively, were dissolved in acetonitrile (conc. 0.025 mg/mL in each case), filled into a glass cuvette and the UV absorption of the resulting solutions measured without and with exposure to UV radiation of a wavelength of 366 nm (HP G1103A 8453 UV-Vis Spectrophotpmeter).

Similarly, for comparison, solutions of the corresponding MALDI matrix compounds (2,5-dihydroxyacetophenone; 2,6-dihydroxyacetophenone and 2-mercaptobenzothiazole, respectively) were dissolved in acetonitrile (conc. 0.025 mg/mL in each case) and the UV absorption of the resulting solutions measured without and with exposure to UV radiation of a wavelength of 366 nm, too.

It was found that the UV absorption spectra of the MALDI matrix compounds 2,5-dihydroxyacetophenone, 2,6-dihydroxyacetophenone and 2-mercaptobenzothiazole did not change upon exposure to UV radiation of a wavelength of 366 nm, indicating that 2,5-dihydroxyacetophenone, 2,6-dihydroxyacetophenone and 2-mercaptobenzothiazole are stable towards UV irradiation at a wavelength of 366 nm.

For the above-stated Photo-caged MALDI Matrix Compounds it was found that their UV absorption maxima were changing upon exposure to UV radiation of a wavelength of 366 nm in a manner approximating the absorption maxima of the corresponding MALDI matrix compound in each case. It can therefore be concluded that the Photoremovable Protecting Group Moiety B is removed from the Photo-caged MALDI Matrix Compound in each case upon irradiation with UV radiation of a wavelength of 366 nm, to release a MALDI matrix compound corresponding to the respective organic MALDI Matrix Compound Moiety A of the Photo-caged MALDI Matrix Compound in each case.

### Example 6: MALDI-MSI analytical method involving Photo-sensitive MALDI Matrix Composites according to the invention

### Example 6.1: MALDI-MSI of hog's brain with Photo-caged MALDI Matrix Compound of Ex. 1.2

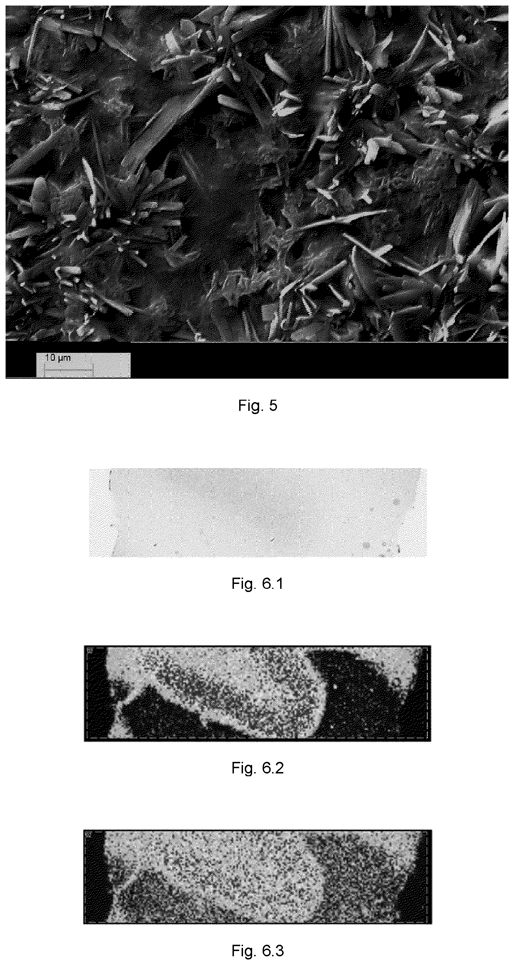

The Photo-caged MALDI Matrix Compound of Ex. 1.2 (DMNB-1,5-DAN) according to the invention was prepared as described in Example 1 above and sprayed onto a sample of sliced hog's brain (analyte; analogously to the method as described in Example 3 above) which was placed on a (glass) object slide. Fig. 6.1 shows a bright field scan of the hog's brain slice covered with Photo-caged MALDI Matrix Compound of Ex. 1.2.

The Photo-sensitive MALDI Matrix Composite according to the invention so received was then analyzed in a known MALDI mass spectrometry imaging (MALDI-MSI) method. Images received from this method show plots of ion intensity vs. relative position of the data from the sample.

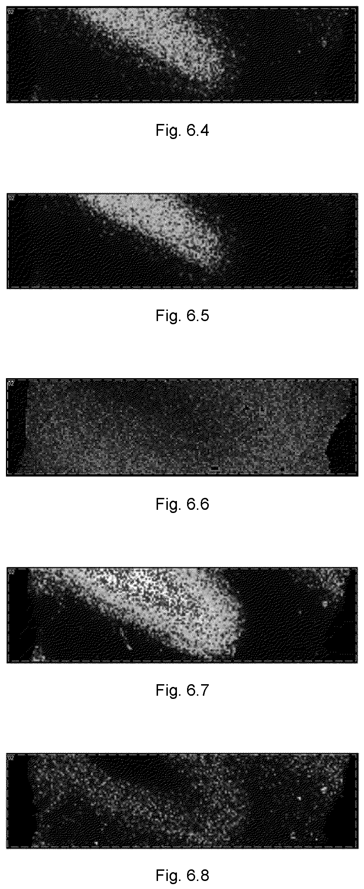

Respective MALDI-MSI ion images at 50 µm spatial resolution in negative ion mode for different mass-to-charge ratios ("m/z-values") are shown in Figures 6.2 to 6.9. The bright areas in Figures 6.2 to 6.9 depict the presence of ions of specific m/z-values (Fig. 6.2: m/z = 726.6; Fig. 6.3: m/z = 728.6; Fig. 6.4: m/z = 750.6; Fig. 6.5: m/z = 766.6; Fig. 6.6: m/z = 788.6; Fig. 6.7: m/z = 885.6; Fig. 6.8: m/z = 888.7; Fig. 6.9: m/z = 904.7). Coloured information from the original MALDI-MSI ion images is not shown in the black-and-white format of Figures 6.2 to 6.9. The mass-to-charge ratio pattern found by the MALDI-MSI method in the sample of sliced hog's brain is typical for a mixture of lipids in animal tissue.

### Example 6.2: MALDI-MSI of hog's brain with Photo-caged MALDI Matrix Compound of Ex. 1.1

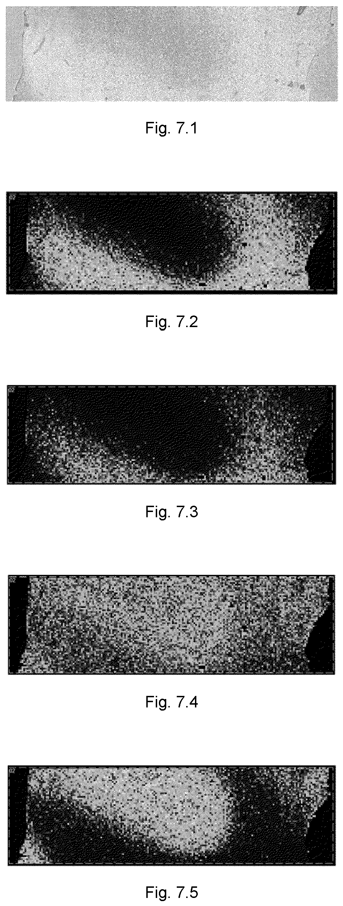

The Photo-caged MALDI Matrix Compound of Ex. 1.1 (DMNB-2,5-DHAP) according to the invention was prepared as described in Example 1 above and sprayed onto a sample of sliced hog's brain (analyte; analogously to the method as described in Example 3 above) which was placed on a (glass) object slide. Fig. 7.1 shows a bright field scan of the hog's brain slice covered with Photo-caged MALDI Matrix Compound of Ex. 1.1.

The Photo-sensitive MALDI Matrix Composite according to the invention so received was then analyzed in a known MALDI mass spectrometry imaging (MALDI-MSI) method as described above for Example 6.1.

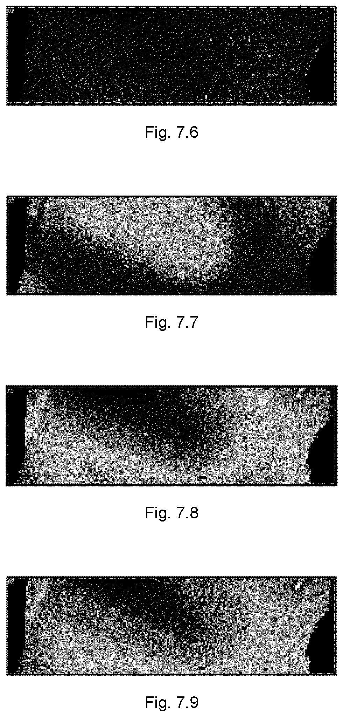

Respective MALDI-MSI ion images at 50 µm spatial resolution in negative ion mode for different mass-to-charge ratios ("m/z-values") are shown in Figures 7.2 to 7.9. The bright areas in Figures 7.2 to 7.9 depict the presence of ions of specific m/z-values (Fig. 7.2: m/z = 726.6; Fig. 7.3: m/z = 728.6; Fig. 7.4: m/z = 750.6; Fig. 7.5: m/z = 766.6; Fig. 7.6: m/z = 788.6; Fig. 7.7: m/z = 885.6; Fig. 7.8: m/z = 888.7; Fig. 7.9: m/z = 904.7). Coloured information from the original MALDI-MSI ion images is not shown in the black-and-white format of Figures 7.2 to 7.9. The mass-to-charge ratio pattern found by the MALDI-MSI method in the sample of sliced hog's brain is typical for a mixture of lipids in animal tissue.

### Example 6.3: MALDI-MSI of hog's brain with Photo-caged MALDI Matrix Compound of Ex. 1.5

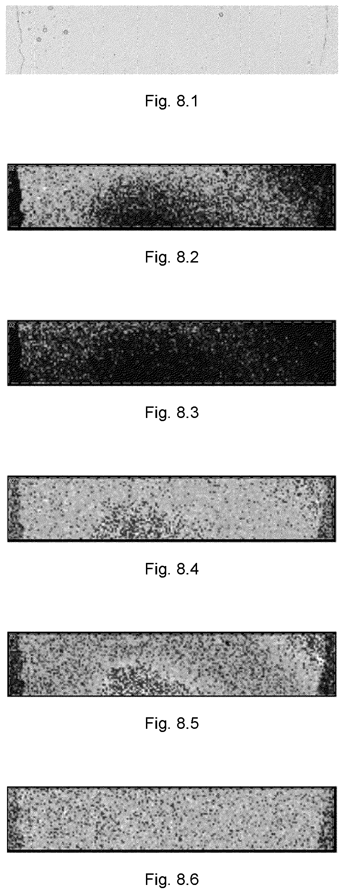

The Photo-caged MALDI Matrix Compound of Ex. 1.5 (DMNB-2,5-DHAP) according to the invention was prepared as described in Example 1 above and sprayed onto a sample of sliced hog's brain (analyte; analogously to the method as described in Example 3 above) which was placed on a (glass) object slide. Fig. 8.1 shows a bright field scan of the hog's brain slice covered with Photo-caged MALDI Matrix Compound of Ex. 1.5.

The Photo-sensitive MALDI Matrix Composite according to the invention so received was then analyzed in a known MALDI mass spectrometry imaging (MALDI-MSI) method as described above for Example 6.1.

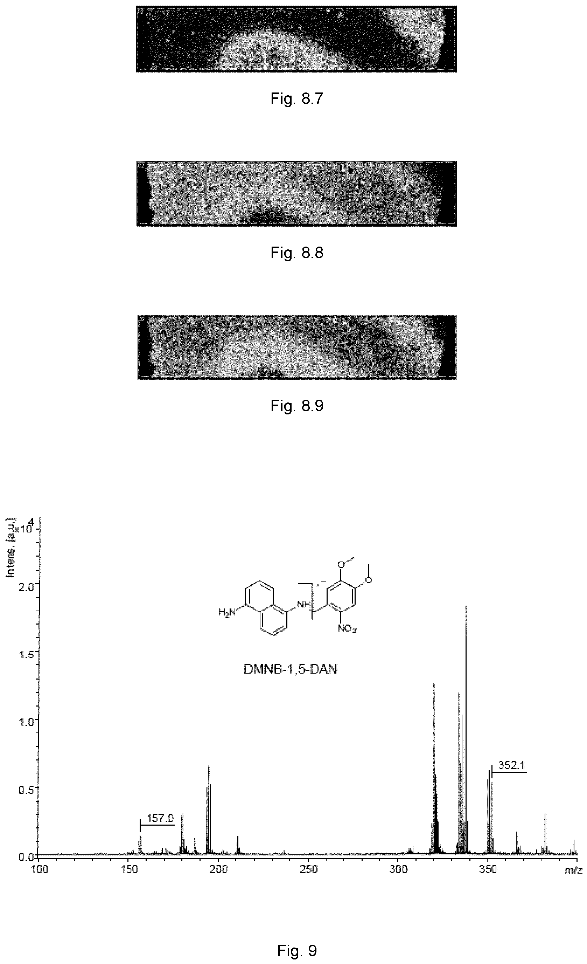

Respective MALDI-MSI ion images at 50 µm spatial resolution in negative ion mode for different mass-to-charge ratios ("m/z-values") are shown in Figures 8.2 to 8.9. The bright areas in Figures 8.2 to 8.9 depict the presence of ions of specific m/z-values (Fig. 8.2: m/z = 726.6; Fig. 8.3: m/z = 728.6; Fig. 8.4: m/z = 750.6; Fig. 8.5: m/z = 766.6; Fig. 8.6: m/z = 788.6; Fig. 8.7: m/z = 885.6; Fig. 8.8: m/z = 888.7; Fig. 8.9: m/z = 904.7). Coloured information from the original MALDI-MSI ion images is not shown in the black-and-white format of Figures 8.2 to 8.9. The mass-to-charge ratio pattern found by the MALDI-MSI method in the sample of sliced hog's brain is typical for a mixture of lipids in animal tissue.

From the results of this Example 6 it can be seen that the Photo-caged MALDI Matrix Compounds according to the present invention are suited for forming Photo-sensitive MALDI Matrix Composites according to the present invention. The Photo-caged MALDI Matrix Compounds according to the present invention and the Photo-sensitive MALDI Matrix Composites according to the present invention promoted desorption/ionization of various lipids from animal tissue.

It could also be shown in this Example 6 that the Photo-caged MALDI Matrix Compounds according to the present invention and the Photo-sensitive MALDI Matrix Composites according to the present invention enable mass spectrometry imaging (MSI) of lipids from animal tissue.

### Example 7: MALDI-TOF MS spectra of Photo-caged MALDI Matrix Compounds according to the invention

### Example 7.1: Photo-caged MALDI Matrix Compound of Ex. 1.2 (DMNB-1,5-DAN)

A MALDI-TOF MS spectrum of the Photo-caged MALDI Matrix Compound of Ex. 1.2 (DMNB-1,5-DAN) was recorded in negative ion mode (see Fig. 9). The fragments of the naphthalene-1,5-diamine radical ion (m/z = 157.0) and the deprotonated molecule ion (m/z = 352.1) could be detected.

### Example 7.2: Photo-caged MALDI Matrix Compound of Ex. 1.1 (DMNB-2,5-DHAP)

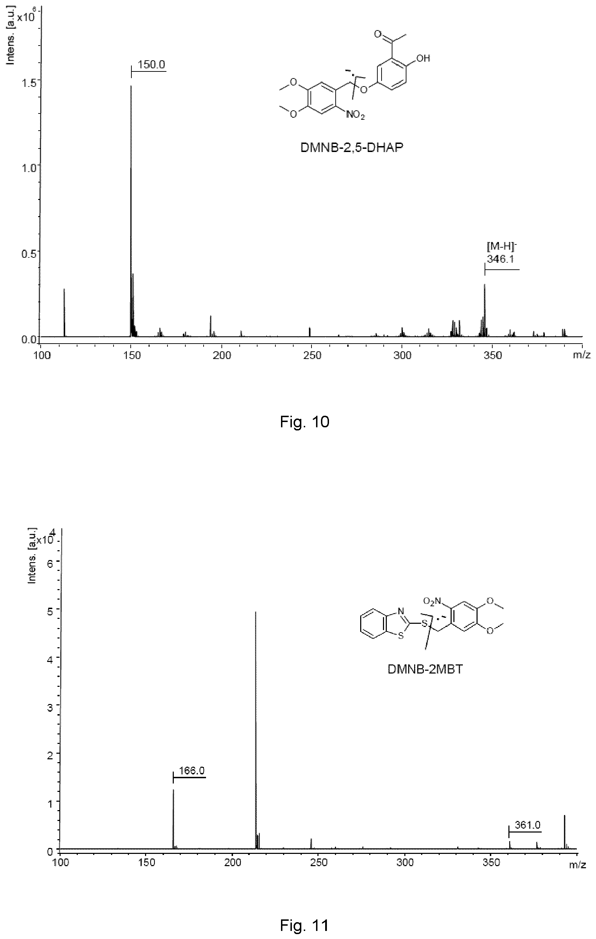

A MALDI-TOF MS spectrum of the Photo-caged MALDI Matrix Compound of Ex. 1.1 (DMNB-2,5-DHAP) was recorded in negative ion mode (see Fig. 10). The fragments of deprotonated 1-(2,5-dihydroxyphenyl)ethan-1-one (m/z = 150.0), 1-(2,5-dihydroxyphenyl) ethan-1-one as radical anion (m/z = 151.0) and the deprotonated molecule ion (m/z = 346.1) could be detected.

### Example 7.3: Photo-caged MALDI Matrix Compound of Ex. 1.5 (DMNB-2MBT)

A MALDI-TOF MS spectrum of the Photo-caged MALDI Matrix Compound of Ex. 1.5 (DMNB-2MBT) was recorded in negative ion mode (see Fig. 11). The fragments of the benzo[d]thiazole-2(3*H*)-thione radical ion (m/z = 166.0) and the deprotonated molecule ion (m/z = 361.0) could be detected.

## Claims

1. Photo-sensitive MALDI Matrix Composite for use in a method of matrix-assisted laser desorption/ionization mass spectrometry, comprising or consisting of
C1) a matrix, completely or partially constituted by a Photo-caged MALDI Matrix Compound, comprising a Photoremovable Protecting Group Moiety B which is covalently bonded to an organic MALDI Matrix Compound Moiety A
and embedded in said matrix
C2) one or more than one analyte, to be analyzed in the method of matrix-assisted laser desorption/ionization mass spectrometry.

2. Photo-sensitive MALDI Matrix Composite according to claim 1,
wherein
- the one or at least one of the more than one analyte is selected from the group consisting of proteins, peptides, lipids, nucleic acids, polysaccharides, glycopeptides, organometallic compounds and organic polymers, wherein preferably the molar mass of the analyte is in the range of from ≥ 100 Da to ≤ 200,000 Da, more preferably of from ≥ 100 Da to ≤ 50,000 Da and even more preferably of from ≥ 100 Da to ≤ 15,000 Da;
and/or
- the total mass ratio of Photo-caged MALDI Matrix Compound : analyte is in the range of from ≥ 100 : 1 to ≤ 20,000 : 1, preferably of ≥ 500 : 1 to ≤ 10,000 : 1;
and/or
- the Photo-sensitive MALDI Matrix Composite is present in the solid state at 23 °C and 101,3 kPa;
and/or
- the Photo-caged MALDI Matrix Compound is photo-cleavable, preferably photo-cleavable at the covalent bond between its moieties A and B, upon irradiation with radiation of a wavelength in the range of from ≥ 100 nm to ≤ 15 µm, preferably of from ≥ 150 nm to ≤ 12 µm, wherein preferably the radiation is or comprises radiation from a laser source, more preferably from a pulsed laser source and even more preferably from a modulated pulsed laser source.

3. Photo-sensitive MALDI Matrix Composite according to any of claims 1 or 2, wherein the Photo-caged MALDI Matrix Compound comprises
M1) an organic MALDI Matrix Compound Moiety A comprising
- two or more than two conjugated double bonds in its chemical structure,
- one or more than one heteroatom in its chemical structure and preferably having
- a molar mass of 500 g/mol or less;
and
M2) a Photoremovable Protecting Group Moiety B,
wherein the Photoremovable Protecting Group Moiety B is covalently bonded to the one or to one of the more than one heteroatoms of the organic MALDI Matrix Compound Moiety A,
wherein preferably the covalent bond between the Photoremovable Protecting Group Moiety B and the one heteroatom, or between the Photoremovable Protecting Group Moiety B and one of the more than one heteroatoms, of the organic MALDI Matrix Compound Moiety A has a bond energy in the range of from ≥ 1 to ≤ 8 eV, preferably in the range of from ≥ 1.5 to ≤ 7 eV.

4. Photo-sensitive MALDI Matrix Composite according to any of claims 1 to 3, wherein the Photoremovable Protecting Group Moiety B of the Photo-caged MALDI Matrix Compound is selected from the group consisting of
- arylcarbonylmethyl groups, preferably selected from the group consisting of phenacyl groups, o-alkylphenacyl groups, p-hydroxyphenacyl groups and benzoin groups;
- nitroaryl groups, preferably nitrophenyl groups;
- coumarin-4-ylmethyl groups;
- arylmethyl groups;
- a pivaloyl group;
- carbonyl cyclo(hetero)alkyl groups;
- carbonylaryl groups;
- arylsulfonyl groups;
- silyl groups;
- 2-hydroxycinnamyl groups;
- α-keto amide groups;
- α,β-unsaturated anilide groups;
- methyl(phenyl)thiocarbamic acid groups;
- thiochromone S,S-dioxide groups;
- 2-pyrrolidino-1,4-benzoquinone groups;
- triazine groups;
- arylmethyleneimino groups;
- xanthene groups
and
- pyronin groups,
or is derived from one of the aforementioned groups;
wherein preferably the Photoremovable Protecting Group Moiety B is selected from the group consisting of
- arylcarbonylmethyl groups, preferably selected from the group consisting of phenacyl groups, o-alkylphenacyl groups, p-hydroxyphenacyl groups and benzoin groups;
and
- nitroaryl groups, preferably nitrophenyl groups.

5. Photo-sensitive MALDI Matrix Composite according to any of claims 1 to 4, wherein the organic MALDI Matrix Compound Moiety A of the Photo-caged MALDI Matrix Compound is selected from the group consisting of
- a moiety of formula I-1 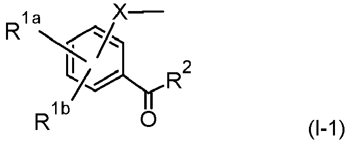 wherein
R^{1a} and R^{1b} are both independently of each other selected from the group consisting of hydrogen, hydroxy, carboxy, methyl and methoxy,
R² is selected from the group consisting of hydroxy and methyl and
X is selected from the group consisting of oxygen, -N(H)-, -N(CH₃)- and -N(C₂H₅)-;
- a moiety of formula I-2 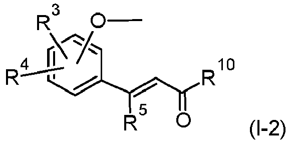 wherein
R³ and R⁴ are independently of each other selected from the group consisting of hydrogen, hydroxy, halogen and methoxy and
R⁵ is selected from the group consisting of hydroxy, methyl and cyano and
R¹⁰ is selected from from the group consisting of hydroxy and methyl;
- a moiety of formula I-3 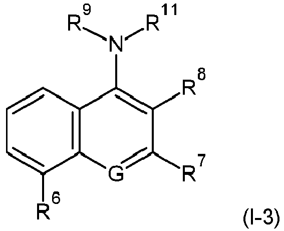 wherein
G is selected from the group consisting of nitrogen and C-H;
R⁶ is selected from the group consisting of hydrogen and amino,
R⁷ and R⁸ are independently of each other selected from the group consisting of hydrogen, amino and methyl, or together with the carbon atoms to which they are bonded, form a saturated or unsaturated five or six-membered ring,
R⁹ is selected from the group consisting of hydrogen, phenyl, saturated branched or unbranched alkyl having 1 to 4 carbon atoms and saturated branched or unbranched aminoalkyl having 1 to 4 carbon atoms and
R¹¹ is a bond or hydrogen,
wherein preferably at least one of the groups G, R⁶, R⁷, R⁸, R⁹ or N-R¹¹ provides a heteroatom, preferably selected from oxygen and nitrogen, to which a Photoremovable Protecting Group Moiety B can be covalently bonded;
and
- a moiety which is derived from a corresponding compound which, upon common chemical modification, provides a respective heteroatom, preferably selected from sulfur, oxygen and nitrogen, to which the Photoremovable Protecting Group Moiety B can be attached, wherein the corresponding compound is selected from the group consisting of 2-mercaptobenzothiazol, dithranol, 3-aminophthalhydrazide, 2,3-dicyanohydroquinone, 1,1'-binaphthyl-2,2'-diamine, 7-aminoquinoline, 2-(4-hydroxyphenylazo)benzoic acid, 6-aza-2-thiothymine, p-nitroaniline, 9-aminoacridine, 2-(2-aminoethylamino)-5-nitropyridine, 1,8-bis(dimethylamino)naphthalene, 7-hydroxycoumarin-4-acetic acid, 7,8-dihydroxy-6-methoxycoumarin, 6,7-dihydroxycoumarin, 7-hydroxycoumarin and 3-hydroxycoumarin.

6. Photo-sensitive MALDI Matrix Composite according to any of claims 1 to 5,
wherein the Photoremovable Protecting Group Moiety B of the Photo-caged MALDI Matrix Compound is a nitrophenyl group, preferably defined by formula II 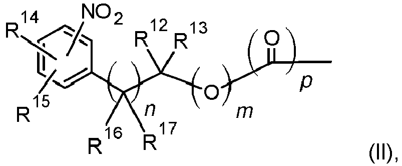 wherein
R¹² is selected from the group consisting of hydrogen and methyl;
R¹³ is selected from the group consisting of hydrogen, methyl and carboxy;
R¹⁴ is selected from the group consisting of hydrogen, methoxy, carboxy, nitro and -O(CH₂)ₗCOOH, wherein I is an integer in the range from 1 to 3;
R¹⁵ is selected from the group consisting of hydrogen and methoxy;
R¹⁶ and R¹⁷ are hydrogen or, together with the carbon atom to which they are bonded, form a carbonyl group; and
m, n and p are independently of each other 0 or 1;
and wherein preferably the MALDI Matrix Compound Moiety A is a moiety of formula I-1 as defined in claim 5.

7. Use of a Photo-caged MALDI Matrix Compound as defined in any of claims 1 to 6
- as matrix compound in a method of matrix-assisted laser desorption/ionization mass spectrometry
and/or
- for preparing a Photo-sensitive MALDI Matrix Composite as defined in any of claims 1 to 6.

8. Use according to claim 7, wherein ≤ 20 % of the total mass of a sample of the Photo-caged MALDI Matrix Compound evaporates after exposure of the sample to a vacuum of ≤ 3300 Pa, preferably of ≤ 10 Pa, for a time period in the range of from ≥ 36 hrs to ≤ 72 hrs, preferably of from ≥ 48 hrs to ≤ 96 hrs, more preferably of from ≥ 72 hrs to ≤ 120 hrs.

9. Matrix-assisted laser desorption/ionization mass spectrometry method, comprising the steps:
S1) providing or preparing a Photo-sensitive MALDI Matrix Composite as defined in any of claims 1 to 6;
S2) irradiating the Photo-sensitive MALDI Matrix Composite as provided or prepared in step S1) with a first radiation of a wavelength suitable to release a MALDI matrix compound from the Photo-caged MALDI Matrix Compound present in the Photo-sensitive MALDI Matrix Composite,
wherein the released MALDI matrix compound has a lower molecular mass and/or a higher vapor pressure at 25 °C than the Photo-caged MALDI Matrix Compound from which it was released,
to receive a MALDI matrix composite, preferably comprising a MALDI matrix compound and, preferably embedded therein, one or more than one analyte to be analyzed;
S3) irradiating the MALDI matrix composite received in step S2) with a second radiation of a nature and wavelength suitable to result in desorption/ionization of the one or more than one analyte from step S2), and preferably also suitable to result in desorption/ionization of the MALDI matrix compound released in step S2),
and
S4) providing the one or more than one desorbed and/or ionized analyte from step S3), and preferably the desorbed and/or ionized MALDI matrix compound from step S3), to a mass spectrometer.

10. Method according to claim 9, wherein the wavelength of the first radiation applied in step S2) and/or the wavelength of the second radiation applied in step S3) are independently of each other in the range of from ≥ 100 nm to ≤ 15 µm, preferably of from ≥ 250 nm to ≤ 10 µm,
wherein preferably
- the first radiation applied in step S2) is or comprises radiation from a laser source, preferably from a pulsed laser source, more preferably from a modulated pulsed laser source,
wherein preferably the wavelength of the radiation is in the range of from ≥ 150 nm to ≤ 700 nm, more preferably of from ≥ 250 nm to ≤ 400 nm;
and/or
- the second radiation applied in step S3) is or comprises radiation from a laser source, preferably from a pulsed laser source, more preferably from a modulated pulsed laser source,
wherein preferably the wavelength of the radiation is in the range of from ≥ 150 nm to ≤ 700 nm, preferably of from ≥ 250 nm to ≤ 400 nm;
and/or
- the first radiation applied in step S2) and the second radiation applied in step S3) both comprise radiation from a pulsed laser source, preferably from a modulated pulsed laser source,
wherein preferably the wavelength of the radiation is in each case in the range of from ≥ 150 nm to ≤ 700 nm, preferably of from ≥ 250 nm to ≤ 400 nm.

11. Method according to any of claims 9 to 10, wherein the first radiation applied in step S2) and the second radiation applied in step S3) are of equal nature and wavelength, wherein preferably
- the irradiation in step S2) and the irradiation in step S3) are performed with
the same radiation,
and/or
- the first radiation applied in step S2) and the second radiation applied in step
S3) are the same;
and/or
- step S2) and step S3) are performed in one single step.

12. Method according to any of claims 9 to 11, wherein step S3) comprises irradiating the one or more than one desorbed but not yet ionized analyte, and preferably the desorbed and/or ionized MALDI matrix compound, with a third radiation from a laser source, preferably from a pulsed laser source, more preferably from a modulated pulsed laser source,
wherein preferably
- the wavelength of the third radiation is in the range of from ≥ 150 nm to ≤ 700 nm, preferably of from ≥ 250 nm to ≤ 400 nm
and/or
- the third radiation is applied to the one or more than one desorbed but not yet ionized analyte when said analyte is in the gas phase, and preferably to the desorbed and/or ionized MALDI matrix compound when said MALDI matrix compound is in the gas phase.

13. Method according to any of claims 9 to 12, wherein a vacuum is applied or maintained in steps S2), S3) and/or S4), wherein preferably
- the vacuum is applied or maintained for a time period of from ≥ 36 hrs to ≤ 72 hrs, preferably of from ≥ 48 hrs to ≤ 96 hrs, more preferably of from ≥ 72 hrs to ≤ 120 hrs;
and/or
- the vacuum comprises a pressure of 3300 Pa or below, more preferably of 5 Pa or below and even more preferably of 0.1 Pa or below.

14. Method according to any of claims 9 to 13, wherein step S1) comprises a step of co-crystallizing a Photo-caged MALDI Matrix Compound as defined in any of claims 1 to 6 with the one or at least one of the more than one analyte as defined in any of claims 1 to 2.

15. Photo-caged MALDI Matrix Compound, as defined in any of claims 1 to 6.
